(19) Europäisches Patentamt
European Patent Office
Office européen des brevets

(11) **EP 3 934 516 B1**

(12) **FASCICULE DE BREVET EUROPEEN**

(45) Date de publication et mention de la délivrance du brevet:
**06.05.2026 Bulletin 2026/19**

(21) Numéro de dépôt: **20725823.7**

(22) Date de dépôt: **04.03.2020**

(51) Classification Internationale des Brevets (IPC):
**A61B 5/00** *(2006.01)* **A61B 1/00** *(2006.01)*
**A61B 5/103** *(2006.01)* **G06T 7/00** *(2017.01)*

(52) Classification Coopérative des Brevets (CPC):
**A61B 5/4244; A61B 1/000094; A61B 1/00188;
A61B 1/042; A61B 1/0669; A61B 1/07;
A61B 1/3132; A61B 5/0082; A61B 5/1032;
A61B 5/413; G06T 7/0014;** A61B 5/6898;
A61B 2576/02; G06T 2207/30056

(86) Numéro de dépôt international:
**PCT/FR2020/050437**

(87) Numéro de publication internationale:
**WO 2020/178526 (10.09.2020 Gazette 2020/37)**

(54) **DISPOSITIF ET PROCÉDÉ D'ÉVALUATION QUALITATIVE DES GREFFONS HÉPATIQUES**

VORRICHTUNG UND VERFAHREN ZUR QUALITATIVEN BEURTEILUNG VON LEBERTRANSPLANTATEN

DEVICE AND METHOD FOR QUALITATIVELY EVALUATING LIVER GRAFTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.03.2019 FR 1902202**

(43) Date de publication de la demande:
**12.01.2022 Bulletin 2022/02**

(73) Titulaire: **Stella Surgical**
**34920 Le Cres (FR)**

(72) Inventeur: **LABICHE, Clément**
**34400 SAINT SERIES (FR)**

(74) Mandataire: **Cornuejols, Georges**
**Cassiopi**
**230 Avenue de l'Aube Rouge**
**34170 Castelnau-le-Lez (FR)**

(56) Documents cités:
**WO-A1-2017/161097     WO-A1-2017/161097
US-A1- 2006 159 325     US-A1- 2006 159 325
US-A1- 2009 262 993     US-A1- 2009 262 993
US-A1- 2012 093 390     US-A1- 2012 093 390**

- **YUNG-NIEN SUN Y N ET AL: "Assessing Liver Tissue Fibrosis with an Automatic Computer Morphometry System", IEEE ENGINEERING IN MEDICINE AND BIOLOGY MAGAZINE, IEEE SERVICE CENTER, PISACATAWAY, NJ, US, vol. 16, no. 3, 1 May 1997 (1997-05-01), pages 66 - 73, XP011084786, ISSN: 0739-5175, DOI: 10.1109/51.585520**
- **YUNG-NIEN SUN Y N ET AL: "Assessing Liver Tissue Fibrosis with an Automatic Computer Morphometry System", IEEE ENGINEERING IN MEDICINE AND BIOLOGY MAGAZINE, IEEE SERVICE CENTER, PISACATAWAY, NJ, US, vol. 16, no. 3, 1 May 1997 (1997-05-01), pages 66 - 73, XP011084786, ISSN: 0739-5175, DOI: 10.1109/51.585520**

**Description**

DOMAINE TECHNIQUE DE L'INVENTION

La présente invention concerne un dispositif et un procédé d'évaluation qualitative de foies humains. Elle concerne, en particulier, le domaine des transplantations de foies entre humains et le traitement de l'obésité.

ÉTAT DE LA TECHNIQUE

Lors des tentatives de transplantation de foie entre humains, il arrive que le foie du donneur présente des altérations qualitatives qui peuvent compromettre la vie du receveur. En particulier, un taux de stéatose supérieur à une certaine valeur limite, par exemple 50 %, est généralement considéré comme incompatible avec une transplantation. Un taux de stéatose inférieur à cette valeur limite mais supérieur à un deuxième valeur limite, par exemple 35 %, impose un dégraissage préliminaire avant une transplantation.

Cependant, l'obtention des données biologiques et la prise de décision, généralement prise par le chirurgien en charge de l'implant, imposent des délais et des frais, notamment de déplacement, qui nuisent au bon déroulement de la transplantation. De plus, l'estimation visuelle n'est pas fiable et le déroulement d'analyses biologiques impose des délais nuisibles à la qualité du foie.

Une évaluation rapide et précise de la stéatose hépatique (SH) par greffe est d'une importance primordiale pour réduire les risques de dysfonction hépatique après une transplantation. L'analyse histopathologique du foie biopsié est l'examen de référence pour évaluer la SH, même si elle est invasive, prend beaucoup de temps et nécessite des outils pas toujours disponibles. En raison du peu de temps disponible entre le prélèvement du foie et la transplantation, les chirurgiens procèdent à l'évaluation de la SH par une évaluation clinique (antécédents médicaux, analyses sanguines) et une analyse visuelle de la texture du foie. Bien que l'analyse visuelle soit reconnue comme un défi dans la littérature clinique, peu d'efforts ont été investis pour développer des solutions assistées par ordinateur pour l'évaluation de la SH.

Concernant le traitement de l'obésité, il n'y a pas de moyens susceptibles de donner immédiatement un diagnostic, notamment un taux de stéatose.

On connait la publication scientifique « Computer-assisted liver-graft steatosis assessment via learning-based texture analysis » de Sara Moccia et al.

OBJET DE L'INVENTION

La présente invention vise à rendre portable et accessible une méthode d'évaluations qualitative de foies humains et à remédier à tout ou partie de ces inconvénients. A cet effet, la présente invention vise, selon un premier aspect, un dispositif d'évaluation qualitative de foies humains, selon la revendication 1.

Grâce à ces dispositions, avant de prélever ou de transplanter le foie, on estime de manière automatique si ce foie est suffisamment sain pour que la transplantation de ce foie soit bénéfique pour le receveur. De même, pour le traitement de l'obésité, on dispose immédiatement d'une estimation objective du taux de stéatose du foie du patient.

Dans des modes de réalisation, le moyen d'estimation est configuré pour appliquer des résultats d'un apprentissage sur des caractéristiques de l'image comportant des nombres de pixels de l'image extraites correspondant à des valeurs de couleurs prédéterminées formant des composantes représentatives de l'image extraite, pour attribuer une évaluation de qualité au foie représenté par l'image captée.

Grâce à ces dispositions, l'évaluation de la qualité du foie est particulièrement précise.

Dans des modes de réalisation, le moyen d'évaluation est configuré pour calculer une valeur représentative de la stéatose du foie, ladite valeur étant une combinaison linéaire de nombres de pixels des valeurs de couleur, appelées composantes, c'est-à-dire à un niveau d'un l'histogramme de l'image pour la composante de couleur, nombres affectés de coefficients multiplicatifs.

Dans des modes de réalisation, le moyen d'estimation est configuré pour utiliser, comme composantes principales, un nombre de composantes supérieures pour les valeurs relatives à la couleur rouge que pour chacune des couleurs verte ou bleue.

Dans des modes de réalisation, les coefficients pour la couleur verte sont, en moyenne, négatifs et de valeur absolue plus importante que pour les autres couleurs.

Dans des modes de réalisation, les coefficients pour la couleur verte ont une moyenne inférieure à la moyenne des coefficients pour les autres couleurs.

Dans des modes de réalisation, les coefficients pour la couleur bleue ont une moyenne supérieure à la moyenne des coefficients pour les autres couleurs.

Dans des modes de réalisation, la majorité des composantes correspond à des niveaux de couleur inférieurs à la moyenne des niveaux de couleur dans les histogrammes.

Dans des modes de réalisation, le nombre de composantes est inférieur à un cinquième du nombre de niveaux de couleurs.

Dans des modes de réalisation, le dispositif objet de l'invention comporte un moyen d'estimation d'un indice de confiance représentatif d'une erreur moyenne pour l'estimation de la santé du foie.

Dans des modes de réalisation, le dispositif objet de l'invention comporte un moyen d'estimation de netteté de l'image captée, à distance du moyen de capture de l'image de foie et un moyen de communication, au moyen de capture d'image, d'un indice de netteté.

Dans des modes de réalisation, le moyen d'estimation de netteté de l'image captée est configuré pour mettre en œuvre un filtrage de Sobel.

Dans des modes de réalisation, le moyen de traitement d'image comporte un moyen de sélection d'une partie d'image.

Dans des modes de réalisation, le dispositif comporte, de plus, un moyen d'introduction, dans le corps du donneur, d'au moins une fenêtre optique du moyen de capture d'une image ainsi que d'une source de lumière pour éclairer le foie du donneur, tout en conservant la stérilité du champ opératoire.

Dans des modes de réalisation, le moyen de traitement est configuré pour détecter d'au moins un reflet sur la surface du foie, dans l'image captée et à extraire de l'image au moins une zone présentant un tel reflet.

Dans des modes de réalisation, le moyen d'estimation de la santé du foie comporte un moyen de réalisation d'un histogramme de couleurs et un moyen de comparaison d'au moins une couleur de cet histogramme avec une couleur normalisée.

Dans des modes de réalisation, chaque couleur normalisée est portée par une charte de couleurs introduite dans le champ de l'image captée.

Dans des modes de réalisation, le moyen d'estimation de la santé du foie comporte un moyen de réalisation d'un échantillonnage de textures et un moyen de comparaison d'au moins une texture avec une texture de référence.

Dans des modes de réalisation, le moyen de capture d'une image est un téléphone mobile.

Dans des modes de réalisation, le moyen de capture d'une image comporte une source de lumière flash et est configuré pour capter une image avec déclenchement de la source de lumière.

Dans des modes de réalisation, le dispositif comporte, de plus, une housse stérile configurée pour contenir le moyen de capture d'image et comportant une fenêtre de prise de vue transparente et rigide à positionner devant l'objectif du moyen de capture d'image.

Dans des modes de réalisation, la housse stérile comporte un filtre polariseur à positionner en regard d'une source de lumière et un filtre polariseur à positionner en regard de l'objectif du moyen de capture d'image.

Selon un deuxième aspect, la présente invention vise un procédé d'évaluation qualitative de foies humains, selon la revendication 15.

Les avantages, buts et caractéristiques particulières de ce procédé étant similaires à ceux du dispositif objet de l'invention, ils ne sont pas rappelés ici.

Dans des modes de réalisation, au cours de l'étape d'affichage, on affiche le résultat calculé dans un intervalle centré sur le résultat et de largeur égale à deux fois l'écart-type issue de la phase d'apprentissage.

Dans des modes de réalisation, le procédé objet de l'invention comporte, de plus, une étape de normalisation des valeurs des histogrammes centrée réduite, avec les valeurs de moyenne et d'écart type issues de la phase d'apprentissage.

BRÈVE DESCRIPTION DES FIGURES

D'autres avantages, buts et caractéristiques de la présente invention ressortiront de la description qui va suivre faite, dans un but explicatif et nullement limitatif en regard des dessins annexés, dans lesquels :

- la figure 1 représente, schématiquement, un premier mode de réalisation d'un dispositif d'évaluation objet de l'invention,
- la figure 2 représente, sous forme d'un schéma-bloc, des fonctions mises en œuvre pour l'évaluation qualitative des greffons de foies pour greffes hépatique,
- la figure 3 représente, schématiquement, un mode de réalisation particulier du dispositif objet de l'invention,
- la figure 4 représente, sous forme d'un logigramme, des étapes d'extraction d'une image d'un foie dans une image captée,
- la figure 5 est un schéma d'un réseau neuronal entièrement convolutif,
- la figure 6 représente un graphique de coefficients de similarité,
- la figure 7 représente des exemples de résultats de segmentation, sous forme de photographies,
- la figure 8 représente des systèmes mis en œuvre dans un mode de réalisation particulier du procédé et du dispositif objets de l'invention,
- la figure 9 représente une courbe de mesure de netteté en fonction de la distance de prise de vue,
- la figure 10 représente, des résultats de mesure de netteté sur des photos de foie humains,
- la figure 11 représente des maques générés automatiquement ou par un opérateur, en regard de photographies de foies,
- la figure 12 représente une courbe de variance en fonction du nombre de composantes étudiées,
- la figure 13 représente, en fonction du nombre de variables, une erreur de classification
- la figure 14 représente des valeurs de coefficients de détermination d'un indicateur de qualité de foie pour quarante variable sélectionnées par apprentissage,
- la figure 15 est un tableau de comparaison des valeurs de stéatose dans trois classes entre la prédiction réalisée par le procédé et le dispositifs objet de l'invention et le résultat observé à la biopsie, à gauche pour les images d'apprentissage et à droite pour les images de prédiction,
- la figure 16 représente un ensemble d'histogramme pour des photographies de foies et la position des variables sélectionnées,
- la figure 17 représente une distribution d'erreurs de prédiction,
- la figure 18 représente des tendances observables selon des représentations,

- la figure 19 représente, sous forme d'un logigramme, des étapes d'un mode de réalisation particulier du procédé objet de l'invention.

## DESCRIPTION D'EXEMPLES DE RÉALISATION DE L'INVENTION

La stéatose hépatique (SH) est l'une des caractéristiques les plus importantes du donneur qui peut influencer la fonction du greffon et donc le résultat de la greffe du foie, aussi appelée, ci-dessous « LT » (pour « liver transplantation »), principalement en raison de lésions d'ischémie et reperfusion lors de la greffe. Définie comme l'accumulation intracellulaire de triglycérides entraînant la formation de vésicules lipidiques dans les hépatocytes, la SH est couramment évaluée par examen histopathologique d'échantillons de tissus hépatiques prélevés par biopsie. En analysant visuellement au microscope les quantités de gouttelettes lipidiques de grande taille de l'échantillon, un score de SH est attribué de manière quantitative en pourcentage (par exemple, 10% ou 30%). Les foies classés comme présentant une infiltration graisseuse de 5 à 30 % sont associés à une diminution de la survie du patient et du greffon, mais ils sont toujours considérés appropriés pour la transplantation en raison de la disponibilité limitée des donneurs. La SH grave (≥ 60%) est plutôt associée à un dysfonctionnement ou à un non-fonctionnement primaire du greffon et n'est pas compatible avec la transplantation.

Bien que l'analyse histopathologique du tissu hépatique biopsié soit actuellement la méthode de référence pour le diagnostic et la classification de la SH dans les greffes de foie, elle est invasive, longue et coûteuse. En raison du peu de temps disponible entre le prélèvement hépatique et la transplantation, le chirurgien procède habituellement à une estimation de la SH par une évaluation clinique (antécédents médicaux, analyses sanguines) et une évaluation visuelle qualitative de la greffe. Dans ce contexte, l'analyse visuelle de la texture du foie est reconnue comme cruciale dans la classification du SH : les foies qui ne peuvent être transplantés en raison d'un SH élevé ont habituellement une texture non homogène et sont plus jaunâtres que ceux qui sont transplantables. Il est néanmoins reconnu que l'estimation précise du SH reste difficile, même entre des mains expérimentées.

Dans ce contexte, l'élaboration d'une méthode robuste, quantitative, pratique, rentable et rapide pour aider le chirurgien à décider s'il doit accepter ou rejeter les greffons de foie est nécessaire. Considérant les défis du diagnostic, des efforts préliminaires à l'évaluation automatisée ou semi-automatisée de la SH ont été proposés et une revue complète peut être trouvée dans la littérature. Par exemple, l'analyse du rapport entre la densité hépatique et splénique a montré une sensibilité (Se) de 79 % dans la reconnaissance du niveau de SH, ou le fibroscan a rapporté une aire sous la courbe de 75

%. L'analyse de l'impédance bioélectrique du foie et la spectroscopie Raman ont été aussi utilisées. Une approche semi-automatique de classement du SH qui exploite la spectroscopie par résonance magnétique (RM) a été proposée, atteignant un coefficient de corrélation de Spearman de 0,90.

Il convient de noter que toutes les méthodes proposées nécessitent des instruments d'imagerie supplémentaires, qui ne sont pas toujours disponibles dans les centres de prélèvements d'organes. De plus, tout au plus, les méthodes ont conclu qu'il existe une corrélation entre les caractéristiques physiques du foie (par exemple, rigidité et impédance du foie) et le grade SH, sans fournir de solution pour l'évaluation de la qualité du greffon hépatique.

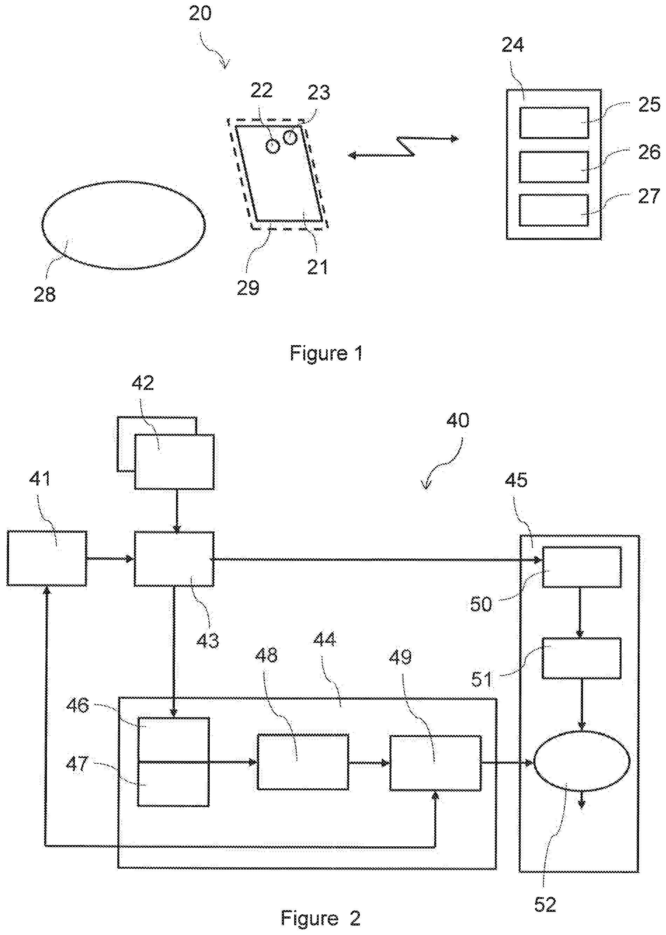

Dans des modes de réalisation, la présente invention vise un dispositif 20 d'évaluation qualitative de foies humains, qui comporte, comme illustré en figure 1 :

- un moyen 21 de capture d'une image de foie humain 28, le foie étant dans le corps du donneur ou déjà prélevé ou mis dans une machine de perfusion de greffon, hypothermique, normothermique et/ou subnormothermique, au moment de la capture de l'image,
- un moyen 25 de traitement d'image configuré pour extraire au moins une partie de l'image du foie depuis l'image captée et
- un moyen 26 d'estimation, à partir de l'image extraite, de la santé du foie.

Selon les modes de réalisation, le foie dont on évalue la qualité est :

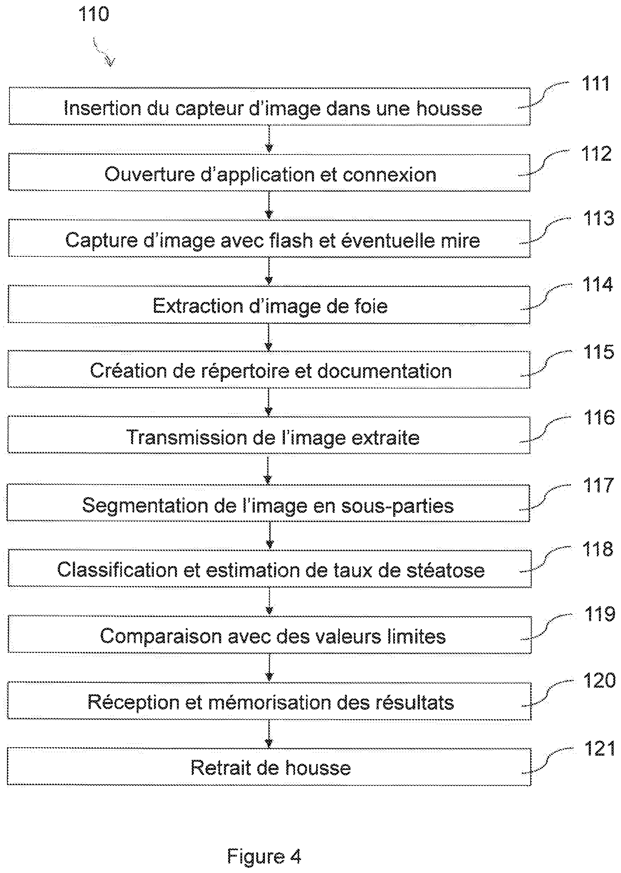

- dans le corps du donneur dont les organes abdominaux sont visibles après incision de la peau, comme illustré en figure 4,
- dans le corps du donneur, le foie étant observé par l'intermédiaire d'un endoscope inséré dans un trocart, l'abdomen du donneur étant gonflé pour pratiquer une coelioscopie où
- déjà extrait du corps du donneur et éventuellement dans une machine de perfusion de greffon.

Dans le mode de réalisation illustré en figure 1, le moyen 21 de capture d'image est un appareil photo numérique ou, préférentiellement, un intelliphone (en anglais smartphone). Ce moyen 21 de capture d'image, muni d'une fenêtre optique 22 sur la lentille frontale de son objectif et d'une source de lumière flash 23, est préférentiellement inséré dans une housse de protection stérile 29.

Les matières utilisées pour la housse peuvent être du polyéthylène ou du polyuréthane ou du silicone avec propriétés tactiles conservées. Des agents antibactériens et fongistatiques peuvent garnir la surface de la housse. La fermeture de la housse se fait par adhésif (système de pli d'un côté de la housse et collage). La housse est compatible avec tous les smartphones dans une plage de dimensions prédéterminée. On note que, avec les matériaux choisis, il n'y a pas de spécificités particulières pour la fenêtre optique positionnée devant le capteur d'images. L'utilisateur plaque la housse sur la fenêtre optique avant de prendre l'image.

Dans des modes de réalisation, la housse stérile 29 comporte un filtre polariseur qui se positionne lors de l'insertion de l'intelliphone en regard d'une source de lumière et un filtre polariseur qui se positionne en regard de l'objectif. Ainsi, dans des modes de réalisation, le dispositif d'évaluation objet de l'invention comporte, de plus, un moyen d'introduction, dans le corps du donneur, d'au moins une fenêtre optique du moyen de capture d'une image, ainsi que d'introduction d'une source de lumière pour éclairer le foie du donneur, tout en conservant la stérilité du champ opératoire.

Dans le cas de l'utilisation de l'endoscope, l'acquisition de la photo se fait uniquement par l'endoscope et non par un smartphone. Cependant :

1) La photo prise par l'endoscope peut être récupérée via une connexion filaire, non filaire (par exemple selon l'un des protocoles Bluetooth, ou wifi, marques déposées) entre la console de récupération des images de l'endoscope et un smartphone pour envoyer les données à un programme implémentant les algorithmes décrits plus loin et avoir immédiatement le résultat concernant le taux de stéatose.

2) Les algorithmes peuvent être intégrés directement dans la console de récupération des images et peut donner un résultat en temps réel du taux de stéatose sur l'écran de celioscopie (aucun smartphone n'est utilisé dans ce cas).

Toutes photos prises avec l'endoscope sont normalisées (balance des blancs en début d'intervention, lumière identique et donc meilleure en termes de qualité).

Dans d'autres modes de réalisation, le moyen de capture d'images comporte des lunettes incorporant un capteur électronique.

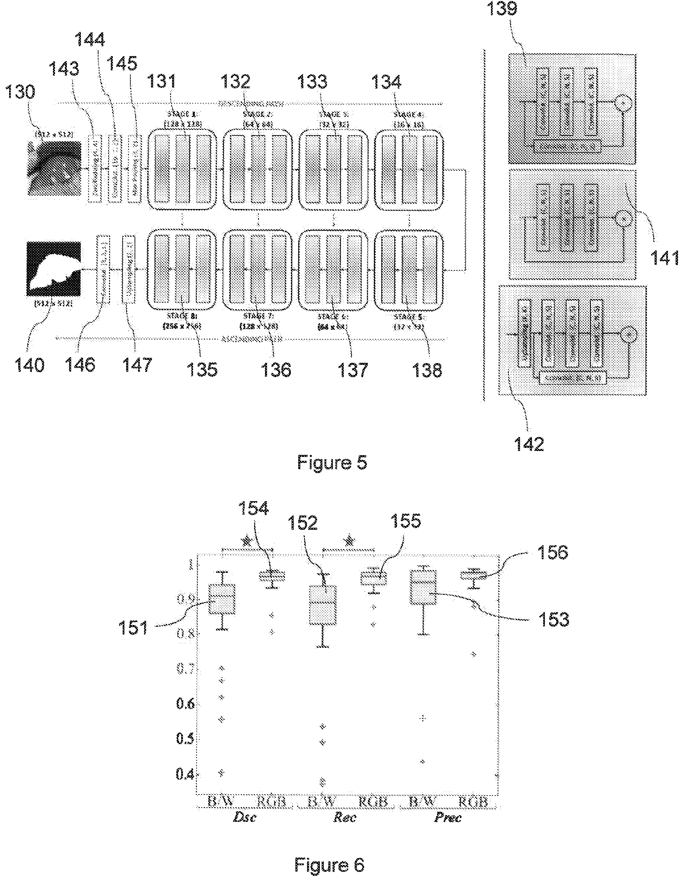

Le moyen 25 de traitement d'image se trouve soit dans le moyen de capture d'image (par exemple dans la console de récupération des images ou dans le smartphone, cas non représenté), soit dans un serveur déporté 24 muni d'une mémoire d'image 27 et d'une unité centrale mettant en œuvre un masque, déterminé automatiquement ou fixe. Un logigramme de masquage automatique est décrit en regard de la figure 5.

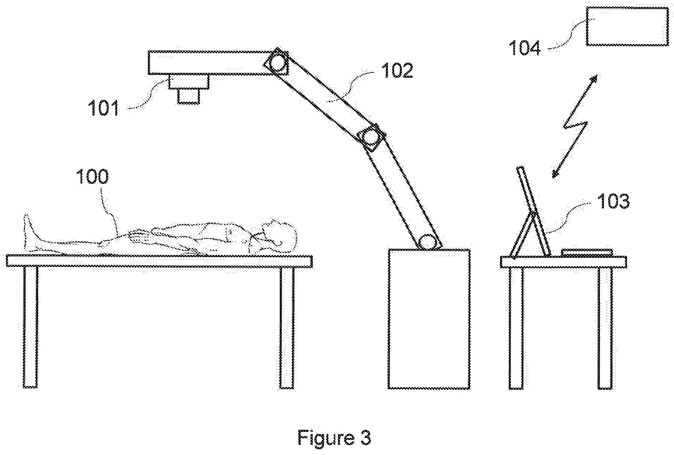

Le moyen 26 d'estimation de la santé du foie se trouve soit dans le moyen de capture d'image, soit dans le serveur déporté 24 muni d'une mémoire d'image 27 et d'une unité centrale mettant en œuvre un algorithme détaillé ci-dessous, notamment en regard des figures 2 et 3.

Grâce à la mise en œuvre du dispositif objet de l'invention, avant de prélever ou de transplanter le foie, on estime si ce foie est suffisamment sain pour que la transplantation de ce foie soit bénéfique pour le receveur. Le chirurgien en charge de la transplantation n'a donc pas besoin de se déplacer ni d'effectuer une évaluation purement visuelle pour accepter le foie ou pour le faire traiter en vue de sa transplantation. De même, pour le traitement de l'obésité, le dispositif fournit au chirurgien une estimation immédiate du taux de stéatose du foie du patient.

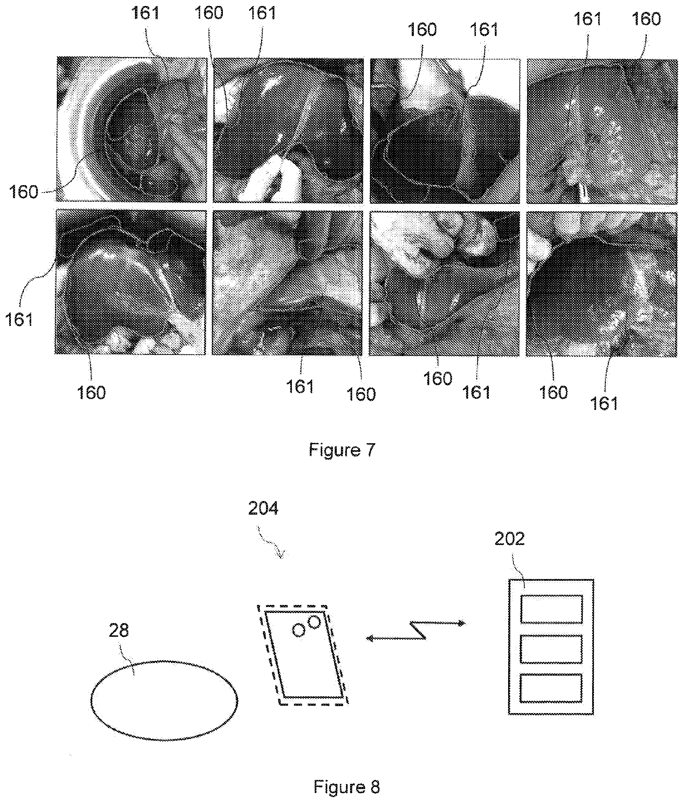

On observe, en figure 8, un système 200 d'assistance à la prise de vue de qualité, c'est-à-dire suffisamment nette et couvrant une zone du foie de dimension supérieure à 6 centimètres et préférentiellement d'au moins les deux tiers du foie soit environ 19 cm en largeur sur 10 cm en hauteur et, encore plus préférentiellement l'intégralité de la partie visible du foie. Ce système 200 comporte un serveur 202 en communication avec un capteur d'images 204, typiquement un téléphone mobile. Préférentiellement, le serveur 202 commande la mise au point de l'objectif du capteur d'image 204 pour avoir une image nette a environ 10 cm. Dans des modes de réalisation, l'autofocus du capteur d'image est laissé libre de réaliser la mise au point.

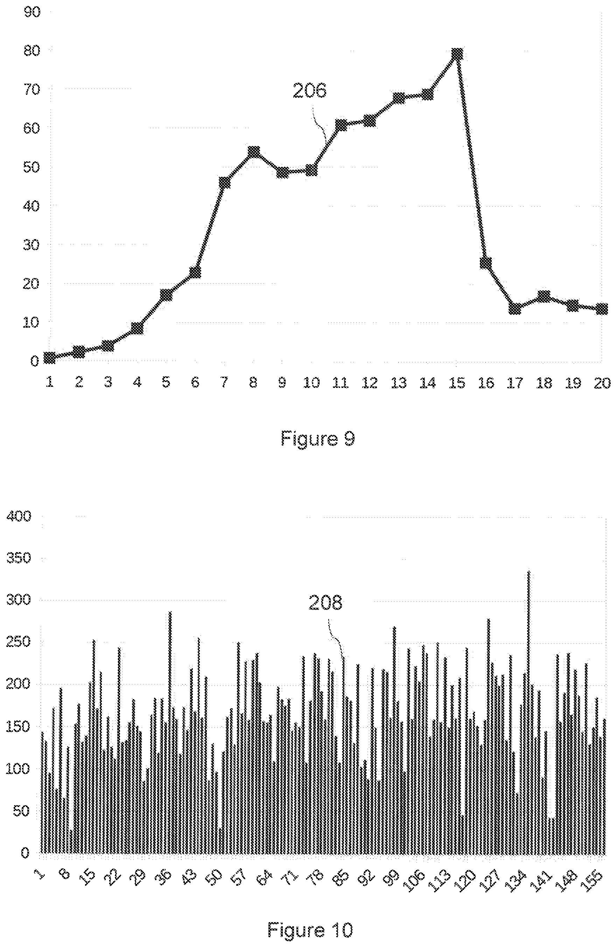

Le serveur 202 reçoit une succession d'images captées par le capteur d'images 204 et détermine si la netteté de chaque image est suffisante pour permettre une évaluation qualitative de foies humains. A cet effet, le serveur 202 détermine le résultat du filtrage de Sobel appliqué à au moins une partie de l'image correspondant au foie ou à l'intégralité de l'image. Le résultat du filtrage de Sobel est comparé à une valeur limite prédéterminée, par exemple 40. La figure 9 représente la courbe des résultats 206 de l'utilisation du filtre de Sobel en fonction de la distance, en centimètres, entre l'objectif du capteur d'image 204 et le foie 28 dont une image est captée. On observe que, aux plus courtes distance, l'image est floue du fait que la distance de mise au point du capteur d'images ne permet pas une prise d'image nette. De plus, aux plus courtes distances, l'image captée ne couvre pas une assez grande portion du foie et n'en présente donc pas de contour. A l'inverse, aux plus longues distances, le flou provient de ce que les détails de l'image de la texture du foie ne sont plus visibles. Entre ces extrêmes, par exemple dans le cas du capteur d'images et de la longueur focale utilisés, entre sept et quinze centimètres, l'image présente l'étendue et la netteté nécessaires au traitement d'image. Si on choisit une valeur limite du filtrage de Sobel égale à 60, les images prises entre onze et quinze centimètres répondent au critère de netteté.

En fonction du résultat de la comparaison de la valeur du filtrage de Sobel et de la valeur limite prédéterminée, le serveur 202 renvoie à une application dédiée installée sur le capteur d'images 204, un message représentatif de la netteté et donc de la capacité à prendre une image. En variante, l'image qui répond à ce critère de netteté est retournée par le serveur 202 au capteur d'images 204 pour y être affichée à son utilisateur, sur un écran.

On note que les algorithmes de filtrage de Prewitt et Roberts donnent aussi une bonne discrimination des images suffisamment nettes. Cependant, l'algorithme de Roberts ont demande plus d'opérations élémentaires.

La figure 10 représente les résultats 208 de l'algorithme de Sobel sur un ensemble de 156 photos de foie humains, après utilisation d'une seule partie de l'image. En effet, les inventeurs ont découvert que, dans les images captées, il y a souvent des tissus unis qui réduisent, pour l'ensemble de l'image, la valeur du filtrage de Sobel.

Dans des modes de réalisation préférentiel, on ne prend en compte qu'une partie de l'image captée, par exemple le quart de l'image présentant la plus haute valeur du filtre de Sobel. Dans ce mode de réalisation, on divise l'image en quatre parties égales, de part et d'autre d'une ligne verticale centrale et de part et d'autre d'une ligne horizontale centrale et on détermine la valeur du filtrage de Sobel pour chacune de ces parties. Puis on ne conserve que la valeur la plus élevée des quatre valeurs obtenues.

On note que, avec les moyens expérimentaux mis en œuvre, la détermination du respect du critère de netteté par le serveur est effectuée en moyenne, en 0,07 seconde par image complète, ce qui permet de retourner une réponse pratiquement instantanée au capteur d'images 204.

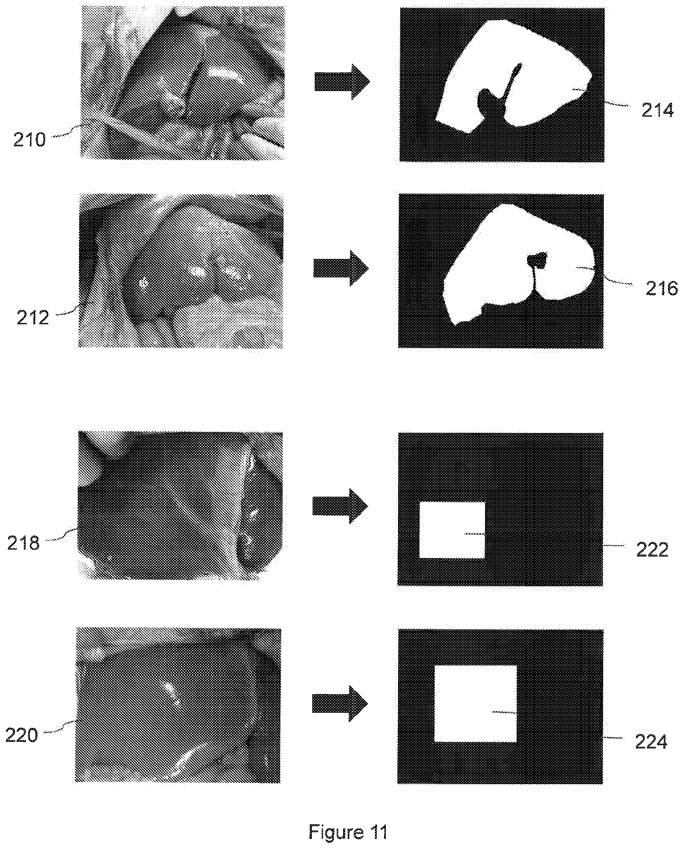

Une fois l'image du foie captée avec le niveau de netteté répondant au critère exposé ci-dessus, un premier masque est appliqué à l'image pour ne pas tenir compte des parties de l'image qui ne représentent pas le foie. A cet effet, dans des modes de réalisation, on réalise une détection automatique de la partie de l'image qui représente le foie, par détourage automatique. Les images de foies 210 et 212, en figure 11, correspondant ainsi aux masques 214 et 216. Dans d'autres modes de réalisation, l'utilisateur, par exemple le chirurgien, positionne un premier masque rectangulaire (ou de toute autre forme prédéterminée) sur l'image captée. Ce premier masque représente une zone d'intérêt que le chirurgien considère être pertinente pour déterminer la qualité du foie. Les images de foies 218 et 220, en figure 11, correspondant ainsi, par exemple, aux premiers masques 222 et 224.

Préférentiellement, on empêche l'utilisateur de sélectionner une zone d'intérêt trop petite et on impose, par exemple, que la zone sélectionnée représente au moins une largeur ou une hauteur supérieure à six centimètres sur le foie. Grâce à ce premier masque dont au moins une dimension est supérieure à six centimètres sur le foie, avec lequel seule la partie représentée en blanc de l'image du foie est conservée pour les traitements ultérieurs, on efface hétérogénéité du foie.

Cette caractéristique de dimensionnement est très différente de cette d'une biopsie qui couvre au maximum deux centimètres. La biopsie ajoute ainsi une étape subjective de choix de la zone de prélèvement. De plus, dans les biopsies, la température (froid) et les stabilisateurs nécessaires à la conservation modifient les résultats et les entachent d'erreur ou, au moins, de variabilité.

On réalise ensuite une élimination de taches blanches dans l'image, qui concernent les reflets, et des ombres. Préférentiellement, pour réaliser cette élimination, on met en œuvre un filtre adaptatif fonction de la valeur moyenne de luminance de l'image ou d'une partie de l'image, pour compenser les différences d'éclairage.

On ne conserve ainsi les valeurs des seuls pixels dont la luminance est supérieure à une première valeur limite prédéterminée, pour éliminer les zones d'ombres, et inférieure à une deuxième valeur limite prédéterminée, par exemple 170 sur chaque canal, pour éliminer les zones blanches.

Dans des variantes, pour l'élimination des ombres, on remplace la valeur de luminance du point par la valeur maximale de luminance des points d'un voisinage entourant le point considéré et on élimine les points dont les valeurs sont, sur chaque canal, inférieures à, par exemple 50 ou 60 sur chaque canal.

Dans ces exemples, on retire ainsi les pixels dont la valeur maximale sur les trois canaux R, V et B est inférieure à 50 ou 60 ou dont la valeur minimale sur les trois canaux est supérieure à 170. Ainsi, un pixel avec une valeur RGB de (207, 22, 75) serait accepté et participerait à la valeur 207 de l'histogramme rouge, à la valeur 22 de l'histogramme vert et à la valeur 75 de l'histogramme bleu.

Les images qui résultent de ces traitements se présentent sous forme de matrices dont certains points, ainsi éliminés, présentent une valeur représentative de leur élimination, par exemple « 0 ». Chaque autre point non éliminé est associé à trois valeurs, par exemple en huit bits, représentatives des canaux rouge, vert et bleu.

Le serveur réalise alors une détermination automatique d'un masque complémentaire, de forme prédéterminée, par exemple circulaire ou elliptique, dont au moins une dimension correspond, sur le foie, à une distance d'au moins six centimètres.

Eventuellement, on effectue une dilatation, par exemple jusqu'à une distance de 15 pixels de long et de 4 pixels de haut. Le masque et la fonction de dilatation sont appliqués sur chaque pixel de l'image. Le but étant de prendre en compte les pixels voisins et donc d'effacer les défauts et les irrégularités de l'image grâce à un phénomène de floutage. Sur l'image, on applique ainsi une fonction de dilatation dans ce masque supplémentaire pour effacer les défauts et leur voisinage.

Enfin, sur l'image résultante, on extrait les histogrammes de valeurs pour chacun des canaux rouge, vert et bleu.

Pour, dans des modes de réalisation, normaliser en intensité les histogrammes des images, site normalisation en luminance, on récupère l'histogramme de chaque canal RGB et la première valeur est supprimée. Les trois histogrammes pour une même image sont mis dans un même vecteur. La somme totale des valeurs présentes dans le vecteur est calculée. Chaque valeur de l'histogramme est divisée par la somme totale.

La normalisation en nombre de pixels est effectuée en divisant chaque nombre de pixel pour un niveau de couleur par le nombre de pixels participant à l'histogramme.

On note que la normalisation en luminance est, dans des modes de réalisation, effectuée avant le seuillage d'extractions de reflets et d'ombres, pour les rendre plus stables. Dans des modes de réalisation, une première normalisation en luminance est effectuée avant seuillage et une deuxième normalisation en luminance est effectuée sur l'image après seuillage.

Les trois histogrammes sont traités de façon indépendante pour constituer un vecteur de trois fois 255 valeurs, soit 765 valeurs. Sur ce triple histogramme, ou vecteur, on applique un lissage en remplaçant, pour chaque niveau de couleur, le nombre de pixels ayant ce niveau de couleur par le nombre de pixels moyen ou médian pour ce niveau et les deux niveaux juste supérieur et juste inférieur, respectivement.

Dans des modes de réalisation, à la place ou en complément de l'utilisation d'un masque supplémentaire, on met en œuvre une détection automatique de points ou de zones d'intérêt, qui consiste à mettre en évidence des zones de cette image jugées « intéressantes » pour l'analyse, c'est-à-dire présentant des propriétés locales remarquables. De telles zones peuvent apparaître, selon la méthode utilisée, sous la forme de points, de courbes continues, ou encore de régions connexes et qui constituent le résultat de la détection.

Après la détection, on applique un algorithme de description qui va se concentrer sur chaque zone d'intérêt détectée pour en calculer des caractéristiques (numériques, en général), (« features » dans la littérature technique en anglais).

La méthode la plus répandue pour le détecter est probablement le détecteur de Harris. Comme celle de Harris, la plupart des autres techniques de détection de points d'intérêt sont fondées sur une analyse locale de l'image à l'ordre 2. Ce qui les différencie entre elles est l'opérateur de dérivation utilisé. On peut citer les méthodes fondées sur l'analyse des DoG (Difference of Gaussians), des LoG (Laplacian of Gaussian) ou des DoH (Difference of Hessians).

Les régions d'intérêt sont des zones d'intérêt plus générales que les points, utiles lorsque les structures recherchées dans une image ne correspondent pas à des points saillants, par exemple, lorsque l'image a subi un lissage important ou lorsque les contours sont épais et progressifs.

Souvent, ces techniques commencent par identifier des points d'intérêt qui vont se révéler être des sortes de barycentres des régions recherchées (blobs en anglais), telles que les méthodes multi-échelles fondées sur l'étude des détecteurs de points d'intérêt cités précédemment (Harris, DoG, etc), à différentes échelles de l'image. Ceci permet d'obtenir des régions soit circulaires soit elliptiques, selon le niveau de raffinement voulu. Ces méthodes sont souvent intégrées à des algorithmes plus généraux tels que SIFT ou SURF, qui incluent un descripteur de région d'intérêt en plus d'un détecteur.

Parmi les détecteurs de régions d'intérêt plus généraux existe également MSER (Maximally Stable Extremal Régions).

On décrit, ci-dessous, des étapes d'apprentissage, puis d'évaluation qualitative de foies humains, de modes de réalisation particuliers de l'invention.

Les composantes (« features ») retenus pour l'apprentissage sont, comme expliqué ci-dessus, les valeurs issues des histogrammes RGB. Sur la base d'un ensemble (par exemple une trentaine) d'images correspondant à des foies dont la qualité a été qualifiée, en termes de stéatose, par des experts et/ou par des techniques connues, telles que les biopsies, on réalise un apprentissage éparse.

La variable y est le résultat de la stéatose prédit par l'algorithme.

$$ y = b0 + \sum_{k=1}^{xV} bk * Xk $$

Formule dans laquelle :

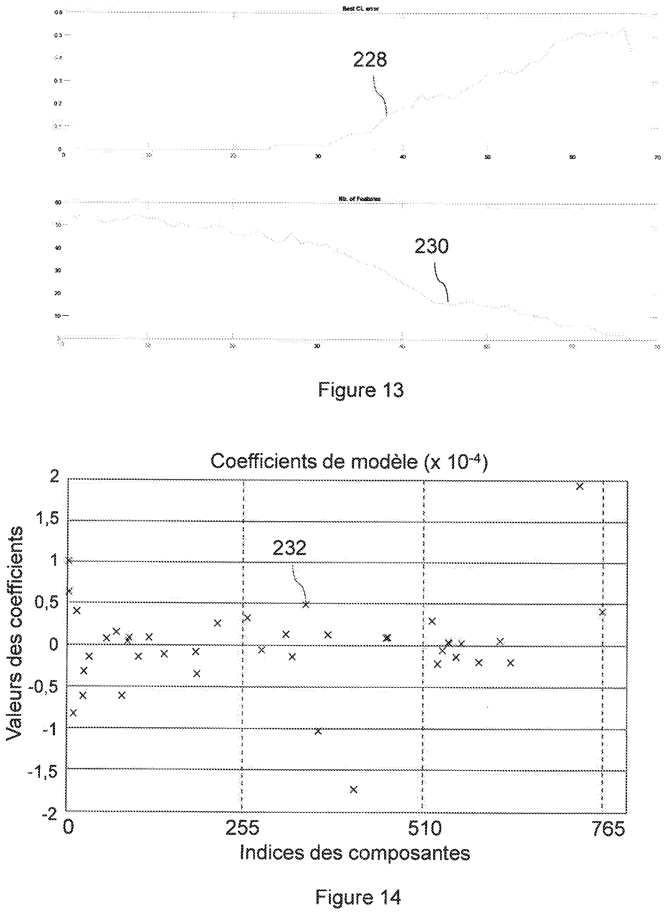

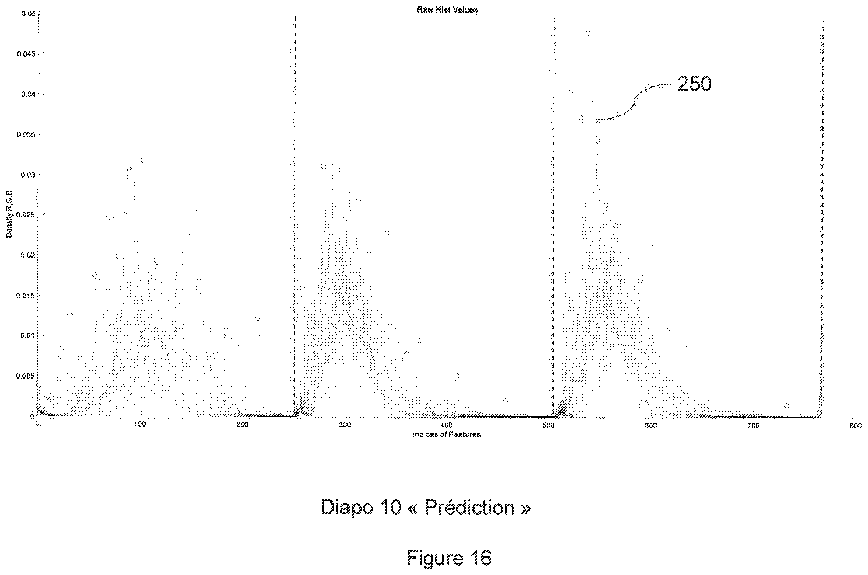

b0 est une valeur fixe (b0 = 28,4 par exemple)
xV est l'ensemble des composantes traitées (ici une quarantaine mais les inventeurs ont constaté qu'avec l'augmentation des exemples dans la base d'apprentissage ce nombre se stabilise autour de 90. Préférentiellement, le nombre de composantes principale choisi est inférieur à un cinquième du nombre de composantes,
bk sont les coefficients multiplicatifs associés aux composantes traitées (voir figure 14) et
Xk est le nombre de pixels de la partie d'image traitée possédant une valeur de couleur correspondant à une composante principale, c'est-à-dire un niveau de l'histogramme de l'image en cours de traitement pour la composante de couleur concernée (voir figure 16).

Celui-ci classe y dans une de trois classes correspondant à des intervalles de valeurs de stéatose [0, 30), [30, 50), [50, 100) comme décrit précédemment.
Ces intervalles sont utilisés pour réaliser le diagnostic final par le chirurgien. Elles permettent de définir si un foie est non stéatosique (sain), y étant dans la plage de valeurs (0 à 30), moyen, y étant dans la plage de valeurs (30 à 50) ou défectueux, correspondant à un risque sur deux de rejet du greffon après transplantation, y étant dans la plage de valeurs (50-100).
b0 représente la prédiction la plus grossière en absence de toute information. C'est simplement la moyenne des valeurs connues de stéatose.

Les facteurs bk sont les résultats de l'algorithme d'apprentissage sur les images utilisées pour cet apprentissage. Bien entendu, un autre algorithme d'apprentissage pourra déterminer d'autres composantes principales et d'autres facteurs à leurs appliquer.

L'homme du métier pourra adapter l'invention, à partir d'une base de données de photos de foie, d'un traitement d'image, suivi de la phase d'apprentissage, retrouver des valeurs de coefficients.

Les valeurs y calculées par la formule approximent des valeurs observées de stéatose dans la plage de valeurs (0, 100). Les valeurs de y inférieures à 0 sont ramenées à 0 et les valeurs supérieures à 100 sont ramenées à 100. Ensuite, le foie est classé dans une des 3 classes selon que la valeur y est dans la plage (0, 30), (30, 50), (50, 100).

L'algorithme a appris sur une base de 33 photos avec biopsies (variables). L'objectif de ce test était de voir le résultat de 54 photos testés sans la biopsie pour comparer ensuite le résultat de la biopsie (référence) avec le résultat de l'algorithme (3 classes).

Dans la première étape d'apprentissage, on prédit y avec le groupe de variables (trois valeurs d'histogrammes sur huit bits, en trois couleurs) en minimisant le nombre de variables retenues. Dans la deuxième étape, on prédit y avec la totalité des variables retenues.

Après avoir comparé le résultat entre la biopsie de référence et le résultat de l'algorithme, on obtient une valeur d'erreur de prédiction.

Dans ce cas, l'algorithme a été appris à partir de 40 variables ou composantes principales.

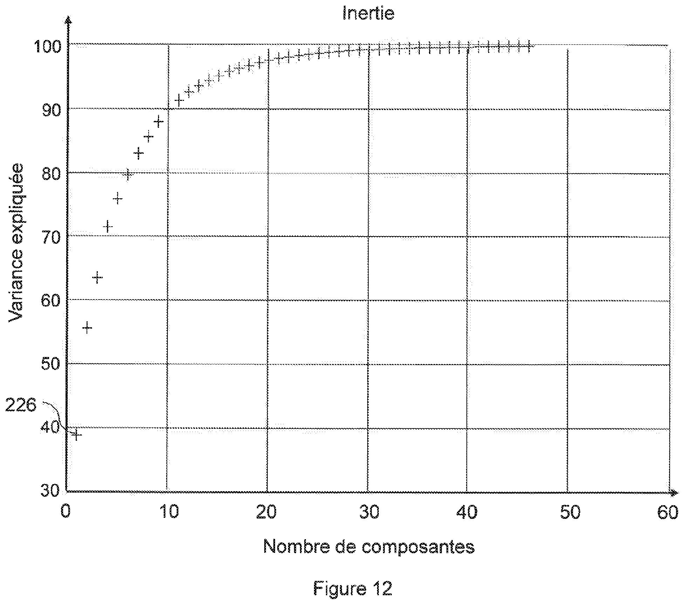

Dans cette étude, on a utilisé entre 30 et 40 variables pour avoir un résultat de plus 95% de prédictions positives selon les trois classes. Pour réduire ce nombre de composantes, on réalise une analyse en composantes principales. On voit, sur la figure 12, 40 composantes (niveaux de couleurs) suffisent pour représenter 99 % de la variance 226.

La figure 12 indique la part de la variance expliquée en fonction du nombre de composantes principales. Avec 30 composantes principales on obtient 98% de la variance expliquée. C'est un indicateur du nombre de features nécessaires pour prédire la stéatose. Il est important de noter que nous faisons la prédiction à partir des features extraites de l'image et pas à partir des composantes principales. Néanmoins, le nombre 40 de features retenues est de même ordre que le nombre 30 des composantes principales.

Concernant l'analyse en composantes principales et le « Sparse Learning », le lecteur pourra se référer aux ouvrages suivants :

- « Analyse en composantes principales », Gilbert Saporta. Probabilités, Analyse des données et statistique. LIVRE, Technip (Editions), 2011.
- "The Elements of Statistical Learning: Data Mining, Inference, and Prediction", Trevor Hastie, Robert Tibshirani, Jerome Friedman - Springer, 2009.
- "Sparse Learning", M. Jiu, N. Pustelnik, S. Janaqi, M. Chebre, P. Ricoux, "Sparse hierarchical interaction learning with epigraphical projection", accepted to Journal of Signal Processing Systems, 2019.

Comme illustré en figure 13, la procédure d'apprentissage éparse diminue le nombre de variables (tableau du bas) et surveillant l'erreur de classification 228 (tableau du haut). Le croisement des deux tendances se produit entre 30 et 40 variables. L'optimum choisi est 40 variables. Lorsque le nombre de features diminue (axe bas) l'erreur de prédiction augmente. Un compromis est trouvé pour 40 variables.

La figure 14 représente les coefficients bk 232 non nuls des 40 variables choisies. Les lignes discontinues verticales montrent les séparations entre les histogrammes correspondant, successivement au couleurs rouge, vert et bleu, de gauche à droite.

On observe que les coefficients bk ont pour valeurs, arrondies à la première décimale, après avoir appliqué un coefficient multiplicatif de 10 000 ($10^4$) :

- pour les composantes dans l'histogramme de la couleur rouge : 1,0 ; 0,6 ; -0,8 ; 0,4 ; -0,6 ; - 0,3 ; -0,1 ; 0,1 ; 0,2 ; -0,6 ; 0,1 ; 0,1 ; -0,2 ; 0,1 ; -0,1 ; -0,1 ; -0,4 ; 0,3 ;
- pour les composantes dans l'histogramme de la couleur verte : 0,4 ; -0,1 ; 0,2 ; -0,2 ; 0,5 ; -1,0 ; 0,2 ; -1,7 ; 0,2 ; 0,2 ;
- pour les composantes dans l'histogramme de la couleur bleue : 0,3 ; -0,2 ; -0,1 ; 0,1 ; 0,1 ; -0,2 ; 0,1 ; -0,3 ; 0,1 ; -0,3 ; 1,9 ; 0,4.

On observe que la moyenne des coefficients pour la couleur rouge est entre 0,0 et 0,05, pour la couleur verte inférieure à - 0,1 et pour la couleur bleue supérieure à 0,1. On observe aussi que la moyenne des valeurs absolues des coefficients pour la couleur rouge est entre 0,3 et 0,35, pour la couleur verte entre 0,45 et 0,5 et, pour la couleur bleue entre 0,32 et 0,37. Ainsi :

- il y a plus de variables et donc de coefficients bk pour la couleur rouge que pour les autres couleurs,
- les coefficients bk pour la couleur verte sont, en moyenne, négatifs et de valeur absolue plus importante que pour les autres couleurs,
- les coefficients bk pour la couleur verte ont une moyenne inférieure à la moyenne des coefficients pour les autres couleurs,
- les coefficients bk pour la couleur bleue ont une moyenne supérieure à la moyenne des coefficients pour les autres couleurs,
- les coefficients concernent plus souvent et avec une valeur absolue plus élevée les niveaux de couleur faible (sombres).

Le dispositif et le procédé de ces modes de réalisation de l'invention effectuent l'évaluation de la qualité du foie en calculant une valeur représentative de la stéatose du foie, ladite valeur y étant une combinaison linéaire de nombres de pixels des valeurs de couleur, appelées composantes, c'est-à-dire à un niveau d'un l'histogramme de l'image pour la composante de couleur, nombres affectés de coefficients multiplicatifs.

Préférentiellement, le moyen d'estimation est configuré pour utiliser, comme composantes principales, un nombre de composantes supérieures pour les valeurs relatives à la couleur rouge que pour chacune des couleurs verte ou bleue.

Dans des modes de réalisation, les coefficients pour la couleur verte sont, en moyenne, négatifs et de valeur absolue plus importante que pour les autres couleurs.

Dans des modes de réalisation, les coefficients pour la couleur verte ont une moyenne inférieure à la moyenne des coefficients pour les autres couleurs.

Dans des modes de réalisation les coefficients pour la couleur bleue ont une moyenne supérieure à la moyenne des coefficients pour les autres couleurs.

Dans des modes de réalisation la majorité des composantes correspond à des niveaux de couleur inférieurs à la moyenne des niveaux de couleur dans les histogrammes.

Dans des modes de réalisation le nombre de composantes est inférieur à un cinquième du nombre de niveaux de couleurs.

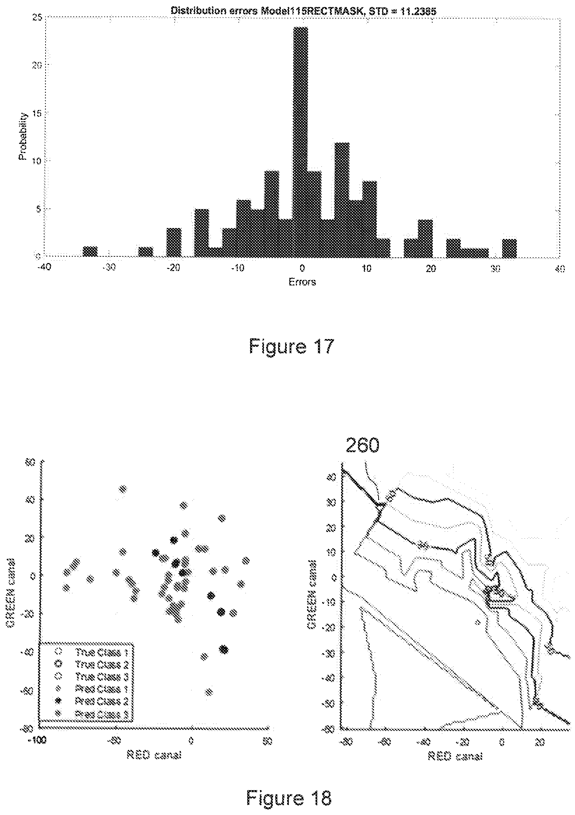

Des tendances s'observent en figure 18. Si l'on calcule la contribution R = somme (bk * xk) sur le canal rouge, la contribution G = somme (bk * xk) sur le canal vert, et B = somme (bk*xk) sur le canal bleu alors, lorsque on dessine les observations sur les axes (R, G), (R, B), (G, B) on voit un gradient de stéatose 260. Les valeurs négatives ou positives sont le résultat de normalisations centrées réduites des valeurs des histogrammes (fonction « zscore » en Matlab).

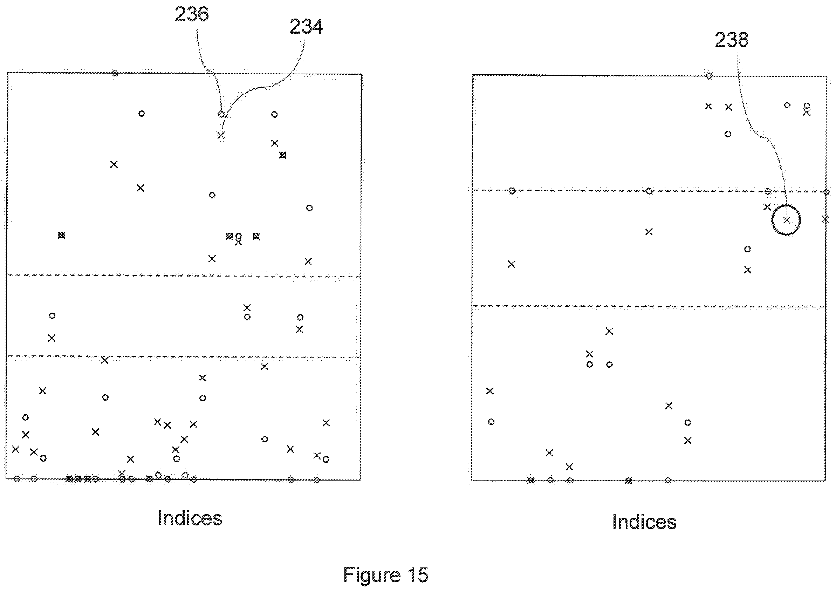

La figure 15 représente le tableau de comparaison des valeurs de stéatose dans les trois classes entre la prédiction 234 et le résultat observé à la biopsie 236, à gauche pour les images d'apprentissage et à droite pour les images de test de prédiction. Le point 238 entouré dans le tableau de droite permet de voir l'erreur de prédiction. Ce point aurait dû être dans la classe « supérieure à 50 » mais il est dans la classe « entre 30 et 50 ». Bien entendu, avec plus d'images de test, notamment représentant des foies avec un taux de stéatose élevé, cette erreur pourra être évitée.

La figure 16 représente l'ensemble des histogrammes traités ainsi que les niveaux de couleurs qui correspondent aux 40 composantes principales. On représente, sur la figure 16, tous les foies sur des histogrammes normalisés, de gauche à droite pour les couleurs rouge, vert et bleu. Les features utilisés sont représentés par des cercles 250.

Une comparaison des canaux RGB peut être réalisée afin de comprendre la tendance des résultats de prédiction, en traçant des lignes de niveau.

Concernant les erreurs de traitement, la figure 17 représente la distribution des erreurs de prédiction. On observe que la majorité des images donnent une erreur de prédiction autour de 12% de stéatose. Ceci donne une alternative de prédiction d'une valeur de stéatose +/- un écart type d'erreur. Ou plus simplement une prédiction en forme d'intervalle (Smin, Smax).

On note que l'écart-type sur la comparaison entre l'évaluation d'un chirurgien expert par analyse visuelle par rapport au résultat de la biopsie par un anapath est de 20 %.

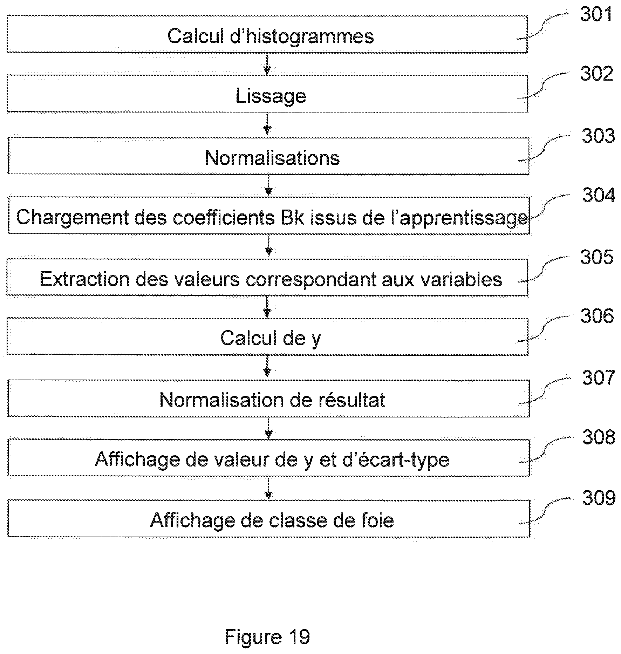

Dans le procédé illustré en figure 19, on observe les étapes suivantes :

- étape 301 : calculer les histogrammes normalisées des canaux R, G, B à partir de l'image. Le résultat est un vecteur x de 255 * 3 = 765 valeurs.
- étape 302 : Lisser les valeurs du vecteur x par une moyenne glissante avec largeur de fenêtre 5 (c'est une variante de la fenêtre 3 exposée ci-dessus),
- étape 303 : Normaliser les valeurs de x par une normalisation centrée réduite avec les valeurs de moyenne et d'écart type retenues dans la phase d'apprentissage à partir de la base d'apprentissage,
- étape 304 : Charger les coefficients b0, bk enregistrés à l'issue de la phase d'apprentissage,
- étape 305 : Extraire les valeurs de x(k), k = 1, ..., m, correspondant aux variables retenues par la phase d'apprentissage ;
- étape 306 : Calculer y = b0 + b1*x (1) + ... + bm * x(m),
- étape 307 : si y < 0 alors y = 0 ; si y > 100 alors y = 100,
- étape 308 : affichage de la réponse [y - s, y + s], s étant l'écart type de l'erreur calculé sur la base d'apprentissage (ici 12 %),

Et/ou

- étape 309 : affichage de la réponse : si y est dans [0, 30) - foie bon (classe 1) ; y est dans [30, 50) - foie à discuter (classe 2) ; y est dans [50, 100) - foie mauvais (classe 3).

On observe, en figure 2, des moyens mis en œuvre dans des modes de réalisation 40 comportant l'apprentissage 44 et l'évaluation de santé 45. Dans un premier temps, comme illustré en partie gauche, des images d'apprentissage 41 précédemment captées comme exposé ci-dessus sont traitées par un moyen 43 de traitement d'image qui extrait au moins une partie de l'image du foie depuis chaque image captée 41. Cette partie d'image du foie est à son tour traitée pour former au moins une sous-image de couleur sensiblement uniforme (préférentiellement, en évitant toute sous-partie d'image représentant un reflet, par exemple une saturation d'au moins une couleur, une ombre, par exemple au moins une couleur étant inférieure à une valeur limite de quelques pourcents de la dynamique du signal, une veine, une fente, ...). Dans la figure 2, deux ensembles 46 et 47 de huit parties d'image, chacun, sont ainsi réalisés. Ces ensembles 46 et 47 de sous-parties sont choisis pour couvrir aussi largement que possible les différentes couleurs et/ou les différentes textures de la partie d'image du foie extraite. De manière équivalente ou pour atteindre cet objectif, dans des modes de réalisation, le moyen 48 réalise un histogramme des couleurs et/ou des textures présentes (à l'exception des reflets ou des ombres de fentes) dans la partie d'image de foie extraite ou une autre extraction de caractéristiques d'image, comme exposé ci-dessous. Un spécialiste, par exemple un laboratoire d'analyse médicale réalisant des biopsies, donne alors une évaluation du taux de stéatose. Eventuellement, un spécialiste, par exemple un chirurgien, donne un avis sur la capacité de greffer le foie et/ou de la dégraisser en vue de sa greffe et/ou sur le traitement de l'obésité à appliquer au patient. Un apprentissage définissant un modèle 49 est alors réalisé. Cet apprentissage peut être réalisé de manière statistique ou avec de l'intelligence artificielle.

Au cours de l'exploitation, une image 42 de foie captée comme exposé ci-dessus suit les mêmes traitements d'images 43. Chaque sous-partie d'image 50 est envoyée au dispositif d'évaluation de santé du foie. L'extraction de caractéristiques 51 correspond à l'extraction de caractéristiques 48. Sur la base du modèle issu de l'apprentissage 49, le moyen d'évaluation 52 de taux de stéatose fournit une valeur de ce taux pour le foie considéré. Eventuellement, le moyen d'évaluation 52 fournit automatiquement un avis sur la capacité de greffer le foie et/ou de la dégraisser en vue de sa greffe et/ou sur le traitement de l'obésité à appliquer au patient.

Dans le mode de réalisation illustré en figure 2 pour l'évaluation de la SH par greffe, à partir de 40 images RGB du foie de 40 donneurs différents, dont la moitié sont des foies acceptés pour la transplantation et la moitié sont des foies refusés, un ensemble de données de sous-parties de tailles 100 × 100 est extrait. A partir de chaque sous-partie, un ensemble de caractéristiques de texture est calculé. Ces caractéristiques sont utilisées pour former un modèle de classificateur. Le modèle formé est utilisé pour évaluer la SH des foies candidats à la transplantation.

En ce qui concerne l'évaluation du taux de stéatose en fonction des caractéristiques des sous-parties d'une image de foie, son algorithme peut être basé sur une analyse de texture basée sur un apprentissage.

On décrit, ci-dessous, des modes de réalisation particuliers, notamment appliqués à l'analyse de la santé de greffons candidats.

Ces modes de réalisation mettent en œuvre une analyse automatique de la texture du foie à l'aide d'algorithmes d'apprentissage automatique pour automatiser le processus d'évaluation de la SH et offrir un soutien au processus décisionnel du chirurgien.

Pour l'apprentissage, au moins quarante images RGB de quarante donneurs différents sont analysées. Les images sont prises à l'aide d'un capteur d'images d'intelliphone (smartphone) dans la salle d'opération. La moitié des images concernent des foies qui ont été acceptés et transplantés et l'autre moitié des images concernent des greffons hépatiques refusés pour la transplantation. Quinze sous-parties d'image du foie choisies au hasard ont été extraites de chaque image, en excluant les zones de reflet et les zones d'ombres. La taille de la sous-partie d'image est, par exemple, de 100 × 100 pixels. De cette façon, un ensemble de données équilibré de 600 correctifs est obtenu. Les caractéristiques fondées sur l'intensité (INT), l'histogramme du modèle binaire local (HLBPriu2) et la matrice de cooccurrence des niveaux de gris (FGLCM) sont examinés. Les caractéristiques de l'échantillon sanguin (Blo) ont également été incluses dans l'analyse. Les approches d'apprentissage supervisé et semi supervisé sont analysées pour la classification des caractéristiques.

Concernant les résultats avec l'ensemble de caractéristiques le plus performant dans ce mode de réalisation (HLBPriu2 + INT+ Blo) et l'apprentissage semi-supervisé, la sensibilité, la spécificité et la précision de la classification sont respectivement de 95%, 81% et 88%.

Cet apprentissage automatique et l'analyse automatique des textures des images RGB provenant de capteurs d'images d'intelliphones permet pour l'évaluation de la SH des greffons. Les résultats montrent qu'il s'agit d'une solution entièrement automatique aidant les chirurgiens dans l'évaluation de la SH dans un bloc opératoire.

On donne, ci-dessous, plus de détails sur cet exemple d'algorithme qui peut être utilisé. La greffe du foie (ou « liver transplantation », dont un acronyme est « LT ») est le traitement de choix pour les patients atteints d'une maladie hépatique terminale pour laquelle il n'existe pas d'autres traitements. En raison de l'augmentation de la demande et de la pénurie d'organes, les critères élargies de sélection des donneurs sont appliqués pour augmenter le nombre de greffes hépatiques. Étant donné que les donneurs à critères élargis génèrent une morbidité et une mortalité accrues dans la population receveuse, l'évaluation de la qualité des greffons hépatique est cruciale.

L'analyse de la texture du foie a l'avantage d'être réalisée sur une image RGB standard, sans nécessiter d'instrumentation supplémentaire. Il convient de noter que les caméras des téléphones cellulaires modernes fournissent des images de qualité élevée pour l'évaluation du foie et sont ubiquitaires.

On explique, ci-dessous, l'approche utilisée dans des modes de réalisation pour l'extraction et la classification des entités texturales. La stratégie d'extraction des caractéristiques est expliquée (section " Extraction des caractéristiques ") ainsi que la formation sur les modèles de classification (section " Formation sur les modèles "). Ces modes de réalisation de l'invention peuvent notamment utiliser les approches de classification supervisées (section " Approches supervisées pour la classification par classes ") et semi-supervisées (section " Approche semi-supervisée pour la classification des images "). Le protocole d'évaluation, qui comprend les matériaux, le paramétrage et la définition des mesures de performance, est expliqué dans la section "Évaluation".

Il convient de noter que la classification par biopsie du pathologiste est associée à l'image entière, et non à une seule sous-partie d'image. Ainsi, le fait de considérer comme pathologiques toutes les sous-parties d'une image d'une greffe présentant une SH élevée peut influencer le résultat de la classification, car la SH n'est généralement pas homogène dans le tissu hépatique. Par conséquent, on examine préférentiellement si la MIL peut soutenir le diagnostic de la SH à partir de sous-parties (non étiquetés) extraites d'images RGB (étiquetées). Parmi les algorithmes MIL, on utilise préférentiellement l'apprentissage à instance unique (SIL), qui a le grand avantage de permettre la fusion de l'information par plages (comme les caractéristiques de texture) avec l'information par images (comme les caractéristiques des échantillons de sang), fournissant ainsi des informations supplémentaires pour le processus de classification. Par exemple, on utilise la formulation populaire de SVM-SIL, qui a montré de bonnes performances de classification.

La SH a été évaluée au moyen d'une analyse histopathologique effectuée après une biopsie du foie.

On observe, en figure 3, un patient, ou donneur 100, positionné sous un moyen de capture d'image 101 soutenu par un bras articulé 102. Un ordinateur 103 reçoit les images captées par le capteur 101 et effectue les traitements décrits ci-dessus, éventuellement en partage avec un serveur distant 104 avec lequel l'ordinateur 103 est en communication.

On note que des lunettes de réalité virtuelle peuvent être mises en œuvre pour assister un opérateur ou un chirurgien pendant la prise et le traitement d'image.

On observe, en figure 4, la succession 110 d'étapes de traitement suivante :

- une étape 111 d'insertion d'un capteur d'image mobile, notamment un intelliphone, dans une housse stérile comportant une fenêtre transparente,
- une étape 112 d'ouverture d'une application et de connexion à un serveur,
- une étape 113 de capture d'image, par exemple en utilisant une source flash et éventuellement une mire de couleurs pour normaliser le signal d'image,
- une étape 114 d'extraction d'au moins une partie d'image du foie, par exemple par masquage manuel, semi-automatique ou automatique, préférentiellement en éliminant les zones de reflets et/ou les zones d'ombre,
- une étape 115 de création d'un répertoire pour le patient ou donneur considéré et d'insertion dans ce répertoire de données d'identification et de données biologiques,

- une étape 116 de transmission de l'image extraite à un serveur distant,
- une étape 117 de segmentation de l'image en sous-parties d'images,
- une étape 118 de classification de l'ensemble des sous-parties d'images pour estimer un taux de stéatose,
- une étape 119 de comparaison du taux de stéatose avec au moins une valeur limite prédéterminée pour décider le traitement à appliquer au foies, notamment, dans le cas d'une transplantation envisagée, décider si le foie est transplantable en l'état, doit être dégraissé ou n'est pas transplantable,
- une étape 120 de réception et de mémorisation des résultats et, éventuellement, d'au moins une partie d'image, dans le répertoire du patient ou du donneur et
- une étape 121 de retrait de la housse stérile.

On décrit, ci-dessous, un algorithme et une méthode de segmentation automatique de foie par acquisition d'images.

Le but est de présenter une solution d'apprentissage profond (« deep learning ») pour la segmentation du greffon à partir d'images de systèmes d'acquisitions acquises au bloc opératoire.

Les simulations ont été effectuées sur trois cent trente-quatre images RGB de différents donneurs et le coefficient Dice de similarité était de 0,9668, le Recall de 0,9685 et la précision de 0,9793. La méthode proposée vise une solution entièrement automatique pour aider les chirurgiens en bloc opératoire.

L'approche basée sur l'analyse de la texture à l'aide de machine à vecteur de support (SVM) pour diagnostiquer la stéatose, en travaillant sur des images RGB obtenues au bloc opératoire a été présentée ci-dessus. Cette méthode, même si elle semble avoir des résultats prometteurs, est limitée en ce qui concerne l'exigence d'une identification manuelle du contour du foie dans l'image.

Le test de référence pour l'identification des contours d'un organe est la segmentation manuelle, mais elle n'est pas adéquate car elle dépend de l'opérateur et est inadaptable dans un contexte particulier comme le bloc opératoire en raison de l'exigence de l'intervention d'un opérateur. De plus, l'utilisation de grandes quantités d'images peut être un process long et fastidieux. Une des stratégies du deep learning qui implique certains filtres convolutifs qui peuvent apprendre hiérarchiquement les caractéristiques de données. Le rôle des filtres consiste à extraire certaines caractéristiques des photos d'entrées et de les collecter dans une cartographie, qui incluent ces fonctionnalités. Le nombre de filtres pour chaque noyau est choisi en fonction du temps nécessaire pour former le réseau et la complexité du problème ; En général, un plus grand nombre de filtres donnera de meilleurs résultats. Cette règle n'est appliquée que jusqu'à un certain seuil car, au-delà de ce seuil, une augmentation du nombre de filtres ne donnera pas plus de performance.

Ci-dessous, on présente une méthode de segmentation automatique d'images RGB prises au bloc opératoire avec la caméra d'un smartphone.

Dans cette étude, un réseau neuronal entièrement convolutif (« FCNN ») a été utilisé. Il est composé de plusieurs noyaux et couches comme le montre la figure 5. Dans le cas présent, le modèle de réseau consiste en réseau convolutif (descendant) et un réseau convolutif ascendant. Le chemin neuronal convolutif commence à effectuer un remplissage zéro (4, 4) ajouter quatre lignes et quatre colonnes de zéros en haut, en bas, à gauche et à droite du tableau d'images. Ensuite, la FCNN continue avec le chemin neuronal convolutif conduit avec seize (7X7) filtres, suivis de la normalisation des lots, d'un filtre linéaire rectifié, d'une activation de l'unité (*ReLU*) et par un regroupement (« pooling ») (3, 3) avec des pas (2, 2). Après ces couches, le chemin descendant continue avec un bloc convolutif composé de trois filtres convolutifs en cascade et une connexion de raccourci avec convolution 1x1. Le bloc convolutif est suivi de deux blocs d'identités convolutives, composés de trois noyaux convolutifs en cascade et une connexion de raccourci d'identité. La combinaison d'un bloc convolutif et de deux blocs convolutifs d'identité est répété quatre fois (figure 5 : de l'étape 131 à l'étape 134). A chaque étape, le nombre de filtres convolutifs par couche est doublé. Le chemin du réseau convolutif ascendant est symétrique à celui de la convolution descendante. Chaque étape, répétée quatre fois (En figure 5 : de l'étape 135 à l'étape 138), présente un bloc de conversion ascendante dans le réseau convolutif. Le chemin ascendant se termine par un bloc de rééchantillonnage de taille (2, 2) et un bloc convolutif, ce cas avec un filtre (3 x 3) et une activation de type sigmoïdienne.

La figure 5 est un schéma du réseau neuronal entièrement convolutif. A gauche, le chemin entier peut être divisé en deux parties : le chemin descendant (en haut) et le chemin ascendant (en bas), chacun composés de quatre étapes. Chaque étape du chemin descendant est constituée d'un bloc convolutif (boîtes à gauche) et de deux blocs d'identification (boîtes au centre et à droite), alors que dans le chemin ascendant, il y a un bloc de conversion ascendante (boîtes à droite) et deux blocs d'identification (au centre et à gauche).

Le bloc « ZeroPadding » (ou marge à « 0 ») 143 représente une couche de remplissage zéro (P, P), avec un remplissage P x P. Le bloc "Convolut." 144 ou 146 représente une couche convolutionnelle (C, N, S), avec les canaux C, la taille du noyau N x N et la foulée S. Chaque couche convolutive est suivie par une couche de normalisation par lots et une Fonction d'activation ReLU. Le bloc "Max Pooling" 145 indique une opération de pooling maximum (N, S) sur N x N patchs et avec stride S. La fonction « UpSampling » 147 désigne une opération de suréchantillonnage (K x K) de taille K. Les flèches verticales en traits discontinus indiquent la concaténation de la carte des caractéristiques à partir du chemin convolutif descendant jusqu'au chemin ascendant. A droite, en 139, 141 et 142, est représenté un exemple de blocs convolutionnels, d'identification et de conversion ascendante.

La figure 6 est une Boxplot (ou « boite à moustaches ») des coefficients de similarité de Dice (Dsc), Recall (Rec) et précision (Prec) pour les niveaux de gris (boxplots 151 à 153) et images en RGB (boxplots 154 à 156). Les étoiles indiquent des différences significatives entre les résultats obtenus avec des images RGB ou celles en niveaux de gris.

Concernant l'entraînement (ou apprentissage), Adam (adaptive momentum estimation) optimizer (marque déposée) a été utilisé pour former le réseau FCNN proposé. Adam a estimé le premier moment $\hat{m}_t$ et le second moment $\hat{v}_t$ du gradient de la fonction de perte (« loss function ») pour mettre à jour un paramètre de réseau e après t mini-lots :

$$\theta_t = \theta_{t-1} - \frac{\alpha}{\sqrt{\hat{v}_t(g_t)} + \varepsilon} \cdot \hat{m}_t(g_t) \tag{1}$$

où $\alpha$ est le pas, *gt* est le gradient par rapport au paramètre e après *t* mini-lots et $\in$ est un petit nombre. La fonction de coût utilisée lors de notre simulation est le coefficient de similarité de Dice où *TP* est le nombre des vrais positifs, *FN* le nombre des faux négatifs et *FP* le nombre des faux positifs. Ces termes sont obtenus à partir des pixels.

Dans le tableau ci-dessous, les médianes des coefficients de similarité de Dice (*Dsc*), recall (*Rec*) et Précision (*Prec*) obtenus pour les images en échelle de niveaux de gris et pour les images RGB. Les fourchettes interquartiles sont indiquées entre parenthèses.

Dsc Rec Prec
Niveaux de gris 0,9102 (0,0835)0,8919 (0,1104)0,9516 (0,0956)
RGB 0,9668 (0,0234)0,9685 (0,0350)0,9793 (0,0191)

$$Dsc = \frac{2 \times TP}{FN + FP + 2 \times TP} \tag{2}$$

La figure 7 présente des exemples de résultats de segmentation. Les lignes 161 et 160 font référence respectivement au masque d'origine en échelle de niveaux de gris et le masque de l'image originale RGB.

On a choisi le meilleur modèle d'analyse sur le *Dsc* de validation, et il a été surveillé pendant l'entrainement. Deux simulations ont été effectuées : dans la première, on a entrainé le FCNN avec des photos en RGB et le second avec des images en niveaux de gris, obtenues en convertissant les photos originales. La principale différence est dans la phase de prétraitement et sont liées aux dimensions des photos où les images du foie sont enregistrées. En particulier, ils se différencient par le nombre de canaux.

Concernant le protocole expérimental, trois cent trente-quatre photos RGB de différents donneurs ont été utilisées. La taille de l'image était 1632 * 1224 pixels. Les images ont été capturées avec la caméra d'un smartphone RGB (12 mégapixels) en intra opératoire. Pour chaque image, une segmentation manuelle du foie a été réalisée, pour séparer le foie du fond de la photo. De cette façon, la segmentation a été obtenue avec traçage manuel des contours du foie.

Le jeu de données (« dataset ») pour l'entrainement était composé de trois cents photos et trois cents masques hépatique manuelles correspondants. Le jeu de données (cohorte de validation) était composé de trente-quatre images différentes. Dans la première simulation, nous avons utilisé des images RGB, alors que dans la seconde les mêmes images étaient converties en niveaux de gris. Pour la première simulation, dans la section relative à la préparation des jeux de données, le pré-traitement des images et des masques ont été réalisées afin d'obtenir un tableau d'images RGB, où chaque image avait trois canaux et un tableau de masques avec un canal. A contrario, pour la deuxième simulation, le prétraitement était plus simple et les images et masques en niveaux de gris ont été construites comme des tableaux avec un seul canal.

Dans cette section, les masques et les images ont été recadrées pour réduire leurs dimensions à 1224 X 1224 pour les rendre de forme carrée. Successivement, les images ont été redimensionnées successivement en 512 X 512, pour réduire la puissance de calcul et la mémoire nécessaire, les proportions ont été maintenues et les images n'étaient pas déformées.

Ensuite, nous avons entrainé la FCNN, initialement avec une taille de lot de 15 sur 100 itérations et un taux d'apprentissage de $10^{-2}$, puis 100 autres itérations avec un lot de 15 et un taux d'apprentissage inférieur égal à $3 \times 10^{-5}$. Un taux d'apprentissage plus élevé était utile pour accélérer la convergence puisqu'une valeur inférieure garantit que nous n'avons pas raté les performances minimales nécessaires. Les 40% des images d'entraînement ont été utilisées comme test de validation.

Les résultats de la segmentation automatique sont comparés à ceux de la segmentation manuelle, le gold standard. Pour évaluer la performance de la segmentation de la proposition FCNN nous avons calculé le *Dsc,* Recall (*Rec*) et la précision (*Prec*) des masques prédits sur MATLAB (marque déposée) :

$$Rec = \frac{TP}{TP + FN}$$

$$Prec = \frac{TP}{TP + FP}$$

Où *TP, FN* et *FP* sont déjà définis. Pour l'entraînement, le test des sommes des rangs de Wilcoxon (niveau de signification égal à 5%) pour des médianes, est utilisé pour évaluer s'il y a des différences statistiques entre le masque prédit à partir de la segmentation RGB des images et de la segmentation des images en niveaux de gris. Toutes les expérimentations sont réalisées sur Google Colaboratory (marques déposées). En revanche, les segmentations manuelles du foie et analyse statistique ont été réalisées sur MATLAB.

Concernant les résultats, une différence significative a été trouvée en comparant le *Dsc* et le *Rec* calculé sur les masques prédictifs dérivés des images RGB et les masques prédictifs dérivés des niveaux de gris. A contrario, aucune différence significative n'a pu être appréciée sur le *Prec.* En particulier, les médianes et les interquartiles de Dsc, Rec et Prec pour les masques prédits, nous les avons déduits de la simulation avec des images RGB en surperformant la simulation avec les niveaux de gris, comme indiqué dans le dernier tableau ci-dessus. Les résultats, présentés dans les boxplots de la figure 6, ont été statistiquement validés avec le test de Wilcoxon. La figure 7 montre un échantillon des résultats de la segmentation où il est possible de comparer les masques prédictifs, dérivés des niveaux de gris et des images RGB avec les masques manuels.

Dans le dernier tableau, il est possible de voir les résultats obtenus avec la segmentation automatique en utilisant les images RGB par rapport à la segmentation automatique utilisant les images en échelle de gris, en termes de *Dsc, Rec* et *Prec.* En particulier, les résultats de *Prec* ne présentent pas de différence significative, basée sur la somme des rangs de Wilcoxon (niveau significatif $\alpha$ égal à 5%), comme le montre la figure 6. Les médianes *Prec* pour les images en niveaux de gris et les images RGB sont respectivement égales à 0,9516 et 0,9793. En revanche, les résultats de *Dsc* et *Rec* montrent des résultats significativement différents. Les médianes *Dsc* pour les images en niveaux de gris et RGB sont respectivement égales à 0,9102 et 0,9668. A contrario, les médianes de *Rec* des images en niveaux de gris et des image RGB sont respectivement égales à 0,8919 et 0,9685.

L'utilisation d'images en niveaux de gris simplifie le modèle en améliorant d'un point de vue clinique, mais les différences significatives excrètent de l'analyse statistique suggèrent que de meilleurs résultats peuvent être réalisés en utilisant des images RGB.

On observe que les moins bonnes prédictions sont obtenues lorsque la portion de foie est petite dans l'image originale. Un autre aspect qui pourrait affecter les prédictions est l'absence d'une claire et nette distinction entre le foie et les tissus environnants. Un exemple de mauvaise prédiction de masque hépatique est montré dans l'échantillon en haut à gauche de la figure 7.

**Revendications**

**1.** Dispositif d'évaluation qualitative de foies humains, **caractérisé en ce qu'**il comporte :

- un moyen (101) de capture d'une image de foie, le foie étant dans le corps du donneur ou déjà prélevé ou mis dans une machine de perfusion de greffon, hypothermique, normothermique et/ou subnormothermique, au moment de la capture de l'image,

- un moyen (103, 104) de traitement d'image configuré pour extraire au moins une partie de l'image du foie depuis l'image captée, le moyen de traitement d'image comporte :

    - un moyen de génération automatique du masque de détourage du foie, par traitement des couleurs et/ou des contours dans l'image captée,

    - un moyen d'application du masque de détourage à l'image captée et

    - le moyen de traitement d'image étant configuré pour détecter au moins un reflet sur la surface du foie, dans l'image captée, et à extraire de l'image au moins une zone présentant un tel reflet ;

    - un moyen d'apprentissage automatique d'une évaluation de qualité du foie en fonction de valeurs de couleurs mesurées dans d'au moins une extraction d'une image captée ;

    - un moyen (103, 104) d'estimation, à partir de l'image extraite, de la santé du foie ;

dans lequel le moyen d'estimation comporte un moyen d'application des résultats de l'apprentissage automatique sur des caractéristiques de l'image comportant des valeurs de couleurs mesurées dans l'image extraite pour attribuer une évaluation de qualité au foie représenté par l'image captée.

2. Dispositif selon la revendication 1, dans lequel le moyen d'estimation est configuré pour appliquer des résultats d'un apprentissage sur des caractéristiques de l'image comportant des nombres de pixels de l'image extraites correspondant à des valeurs de couleurs prédéterminées formant des composantes représentatives de l'image extraite, pour attribuer une évaluation de qualité au foie représenté par l'image captée.

3. Dispositif selon l'une des revendications 1 ou 2, dans lequel le moyen d'évaluation est configuré pour calculer une valeur représentative de la stéatose du foie, ladite valeur étant une combinaison linéaire de nombres de pixels des valeurs de couleur, appelées composantes, c'est-à-dire à un niveau d'un l'histogramme de l'image pour la composante de couleur, nombres affectés de coefficients multiplicatifs.

4. Dispositif selon la revendication 3, dans lequel le moyen d'estimation est configuré pour utiliser, comme composantes principales, un nombre de composantes supérieures pour les valeurs relatives à la couleur rouge que pour chacune des couleurs verte ou bleue.

5. Dispositif selon l'une des revendications 3 ou 4, dans lequel

    les coefficients pour la couleur verte sont, en moyenne, négatifs et de valeur absolue plus importante que pour les autres couleurs, les coefficients pour la couleur verte ont une moyenne inférieure à la moyenne des coefficients pour les autres couleurs et/ou les coefficients pour la couleur bleue ont une moyenne supérieure à la moyenne des coefficients pour les autres couleurs.

6. Dispositif selon l'une des revendications 3 à 5, dans lequel la majorité des composantes correspond à des niveaux de couleur inférieurs à la moyenne des niveaux de couleur dans les histogrammes.

7. Dispositif selon l'une des revendications 1 à 6, qui comporte un moyen d'estimation de netteté de l'image captée, à distance du moyen de capture de l'image de foie et un moyen de communication, au moyen de capture d'image, d'un indice de netteté.

8. Dispositif selon la revendication 7, dans lequel le moyen d'estimation de netteté de l'image captée est configuré pour mettre en œuvre un filtrage de Sobel.

9. Dispositif selon l'une des revendications 1 à 8, dans lequel le moyen de traitement d'image comporte un moyen de sélection d'une partie d'image.

10. Dispositif selon l'une des revendications 1 à 9, qui comporte, de plus, un moyen d'introduction, dans le corps du donneur, d'au moins une fenêtre optique du moyen de capture d'une image ainsi que d'une source de lumière pour éclairer le foie du donneur, tout en conservant la stérilité du champ opératoire.

11. Dispositif selon l'une des revendications 1 à 10, dans lequel le moyen de traitement est configuré pour détecter au moins un reflet sur la surface du foie, dans l'image captée, et à extraire de l'image au moins une zone présentant un tel reflet.

12. Dispositif selon l'une des revendications 1 à 11, dans lequel le moyen d'estimation de la santé du foie comporte un moyen de réalisation d'un histogramme de couleurs et un moyen de comparaison d'au moins une couleur de cet histogramme avec une couleur normalisée.

13. Dispositif selon l'une des revendications 1 à 12, dans lequel le moyen d'estimation de la santé du foie comporte un moyen de réalisation d'un échantillonnage de textures et un moyen de comparaison d'au moins une texture avec une texture de référence.

14. Dispositif selon l'une des revendications 1 à 13, qui comporte, de plus, une housse stérile configurée pour contenir le moyen de capture d'image et comportant une fenêtre de prise de vue transparente et rigide à positionner devant l'objectif du moyen de capture d'image.

15. Procédé d'évaluation qualitative de foies humains, **caractérisé en ce qu'**il comporte :

   - une phase d'apprentissage de coefficients et de variables en fonction d'histogrammes normalisées des canaux de couleur à partir d'une partie d'une photographie d'un foie et
   - une phase d'évaluation qualitative de foies humains qui comporte :

      - une étape de génération automatique du masque de détourage du foie, par traitement des couleurs et/ou des contours dans une image captée, configurée pour détecter au moins un reflet sur la surface du foie, dans l'image captée, et à extraire de l'image au moins une zone présentant un tel reflet;

   - une étape d'application du masque de détourage à l'image captée et
   - une étape (301) de calcul d'histogrammes normalisées des canaux de couleur à partir d'une partie détourée d'une photographie d'un foie,
   - une étape (304) de chargement de coefficients obtenus à l'issue de la phase d'apprentissage,
   - une étape (305) d'extraction de valeurs des histogrammes correspondant à des variables retenues à l'issue de la phase d'apprentissage ;
   - une étape (306) de calcul d'une combinaison linéaire des valeurs extraites affectées des coefficients chargés et
   - une étape (308, 309) d'affichage d'une information représentative du résultat du calcul de la combinaison linéaire.

**Patentansprüche**

1. Vorrichtung zur qualitativen Bewertung menschlicher Lebern, **dadurch gekennzeichnet, dass** sie Folgendes umfasst:

   - ein Mittel (101) zur Bildaufnahme der Leber, wobei sich die Leber zum Zeitpunkt der Bildaufnahme im Körper des Spenders befindet oder bereits entnommen oder in eine hypothermische, normothermische und/oder subnormothermische Transplantat-Perfusionsmaschine eingebracht wurde,
   - ein Mittel (103, 104) zur Behandlung der Bildaufnahme, das so konfiguriert ist, dass es mindestens einen Teil der Bildaufnahme der Leber aus der aufgenommenen Bildaufnahme extrahiert, wobei das Mittel zur Behandlung der Bildaufnahme Folgendes umfasst:

      - ein Mittel zur automatischen Erzeugung der Ausschnittmaske der Leber durch Verarbeitung der Farben und/oder Konturen in der Bildaufnahme,
      - ein Mittel zur Anwendung der Ausschnittmaske auf die Bildaufnahme
      - wobei das Mittel zur Behandlung der Bildaufnahme so konfiguriert ist, dass es mindestens eine Reflexion auf der Oberfläche der Leber in der Bildaufnahme erkennt und mindestens einen Bereich mit einer solchen Reflexion aus der Bildaufnahme extrahiert;
      - ein Mittel zum automatischen Lernen einer Qualitätsbewertung der Leber auf der Grundlage von Farbwerten, die in mindestens einer Extraktion einer Bildaufnahme gemessen wurden;
      - ein Mittel (103, 104) zur Schätzung der Gesundheit der Leber anhand der extrahierten Bildaufnahme;

   wobei das Mittel zur Schätzung ein Mittel zur Anwendung der Ergebnisse des automatischen Lernens auf Merkmale der Bildaufnahme umfasst, die in der extrahierten Bildaufnahme gemessene Farbwerte umfassen, um der durch die Bildaufnahme dargestellten Leber eine Qualitätsbewertung zuzuweisen.

2. Vorrichtung nach Anspruch 1, wobei das Mittel zur Schätzung so konfiguriert ist, dass es Ergebnisse eines Lernvorgangs auf Merkmale der Bildaufnahme anwendet, die extrahierte Pixelzahlen der Bildaufnahme umfassen, die vorbestimmten Farbwerten entsprechen, die repräsentative Komponenten der extrahierten Bildaufnahme bilden, um der durch die Bildaufnahme dargestellten Leber eine Qualitätsbewertung zuzuweisen.

3. Vorrichtung nach einem der Ansprüche 1 oder 2, wobei das Mittel zur Bewertung so konfiguriert ist, dass es einen Wert berechnet, der für die Lebersteatose repräsentativ ist, wobei dieser Wert eine lineare Kombination von Pixelzahlen der Farbwerte, sogenannten Komponenten, d. h. auf einer Ebene eines Histogramms der Bildaufnahme für die Farbkomponente, ist, die mit Multiplikationskoeffizienten versehen sind.

**4.** Vorrichtung nach Anspruch 3, wobei das Mittel zur Schätzung so konfiguriert ist, dass sie als Hauptkomponenten eine größere Anzahl von Komponenten für die Werte in Bezug auf die Farbe Rot als für jede der Farben Grün oder Blau verwendet.

**5.** Vorrichtung nach einem der Ansprüche 3 oder 4, wobei

die Koeffizienten für die Farbe Grün im Durchschnitt negativ sind und einen größeren Absolutwert als für die anderen Farben haben,
die Koeffizienten für die Farbe Grün einen Durchschnittswert haben, der kleiner ist als der Durchschnittswert der Koeffizienten für die anderen Farben, und/oder
die Koeffizienten für die Farbe Blau einen Durchschnittswert haben, der größer ist als der Durchschnittswert der Koeffizienten für die anderen Farben.

**6.** Vorrichtung nach einem der Ansprüche 3 bis 5, wobei die Mehrheit der Komponenten Farbwerten entspricht, die unter dem Durchschnitt der Farbwerte in den Histogrammen liegen.

**7.** Vorrichtung nach einem der Ansprüche 1 bis 6, die ein Mittel zur Bewertung der Schärfe der Bildaufnahme in einiger Entfernung vom Mittel zur Bildaufnahme der Leber und ein Mittel zur Übermittlung eines Schärfeindexes an das Mittel zur Bildaufnahme umfasst.

**8.** Vorrichtung nach Anspruch 7, wobei das Mittel zur Schätzung der Schärfe der Bildaufnahme so konfiguriert ist, dass es eine Sobel-Filterung durchführt.

**9.** Vorrichtung nach einem der Ansprüche 1 bis 8, wobei das Mittel zur Behandlung der Bildaufnahme eine Vorrichtung zur Auswahl eines Bildausschnitts umfasst.

**10.** Vorrichtung nach einem der Ansprüche 1 bis 9, die zusätzlich ein Mittel zum Einführen mindestens eines optischen Fensters des Mittels zur Bildaufnahme sowie einer Lichtquelle zur Beleuchtung der Leber des Spenders in den Körper des Spenders umfasst, wobei die Sterilität des Operationsfeldes erhalten bleibt.

**11.** Vorrichtung nach einem der Ansprüche 1 bis 10, wobei das Mittel zur Behandlung so konfiguriert ist, dass es mindestens eine Reflexion auf der Oberfläche der Leber in der Bildaufnahme erkennt und mindestens einen Bereich mit einer solchen Reflexion aus der Bildaufnahme extrahiert.

**12.** Vorrichtung nach einem der Ansprüche 1 bis 11, wobei das Mittel zur Schätzung der Lebergesundheit ein Mittel zur Erstellung eines Farbhistogramms und ein Mittel zum Vergleichen mindestens einer Farbe dieses Histogramms mit einer normierten Farbe umfasst.

**13.** Vorrichtung nach einem der Ansprüche 1 bis 12, wobei das Mittel zur Schätzung der Lebergesundheit ein Mittel zur Durchführung einer Texturprobenahme und ein Mittel zum Vergleichen mindestens einer Textur mit einer Referenztextur umfasst.

**14.** Vorrichtung nach einem der Ansprüche 1 bis 13, die zusätzlich eine sterile Hülle umfasst, die so konfiguriert ist, dass sie das Mittel zur Bildaufnahme enthält, und die ein transparentes und starres Aufnahmefenster umfasst, das vor dem Objektiv des Mittels zur Bildaufnahme positioniert werden kann.

**15.** Verfahren zur qualitativen Bewertung menschlicher Lebern, **dadurch gekennzeichnet, dass** es Folgendes umfasst:

- eine Phase des Lernens von Koeffizienten und Variablen in Abhängigkeit von genormten Histogrammen der Farbkanäle anhand eines Teils einer Bildaufnahme einer Leber und
- eine Phase der qualitativen Bewertung menschlicher Lebern, die Folgendes umfasst:

- einen Schritt der automatischen Erzeugung der Ausschnittmaske der Leber durch Verarbeitung der Farben und/oder Konturen in einer Bildaufnahme, der so konfiguriert ist, dass er mindestens eine Reflexion auf der Oberfläche der Leber in der Bildaufnahme erkennt und mindestens einen Bereich mit einer solchen Reflexion aus der Bildaufnahme extrahiert;

- einen Schritt zur Anwendung der Ausschnittmaske auf die Bildaufnahme und
- einen Schritt (301) zur Berechnung genormter Histogramme der Farbkanäle aus einem ausgeschnittenen Teil einer Bildaufnahme einer Leber,
- einen Schritt (304) zum Laden von Koeffizienten, die am Ende der Lernphase erhalten wurden,
- einen Schritt (305) zur Extraktion von Werten aus den Histogrammen, die den am Ende der Lernphase ausgewählten Variablen entsprechen;
- einen Schritt (306) zur Berechnung einer linearen Kombination der extrahierten Werte, denen die geladenen Koeffizienten zugewiesen sind, und
- einen Schritt (308, 309) zur Anzeige einer Information, die das Ergebnis der Berechnung der linearen Kombination darstellt.

## Claims

1. Device for qualitatively evaluating human livers, **characterised in that** it comprises:

   - a means (101) for capturing an image of a liver, the liver being in the donor's body, already collected, or placed in a hypothermic, normothermic and/or subnormothermic graft perfusion machine, at the time the image was captured;
   - an image processing means (103, 104) configured to extract at least one portion of the image of the liver from the captured image, which image processing means comprises:

      - a means for automatically generating a cropping mask for the liver, by processing colours and/or contours in the captured image;
      - a means for applying the cropping mask to the captured image; and
      - the image processing means being configured to detect at least one reflection on the surface of the liver, in the captured image, and to extract from the image at least one area having such a reflection;
      - a machine-learning means for evaluating the quality of the liver on the basis of the colour values measured in at least one extraction of a captured image;
      - a means (103, 104) for estimating the health of the liver, based on the extracted image;

   wherein the estimation means comprises a means for applying the results of the machine learning operation to the characteristics of the image containing colour values measured in the extracted image so as to assign a quality evaluation to the liver represented by the captured image.

2. Device according to claim 1, wherein the estimation means is configured to apply the results from a learning operation to the characteristics of the image comprising the extracted image pixel numbers corresponding to predetermined colour values forming features representative of the extracted image, so as to assign a quality evaluation to the liver represented by the captured image.

3. Device according to one of claims 1 or 2, wherein the evaluation means is configured to calculate a value representative of steatosis of the liver, said value being a linear combination of pixel numbers for colour values, called features, i.e. at a level of a histogram of the image for the colour feature, assigned multiplication coefficient numbers.

4. Device according to claim 3, wherein the estimation means is configured to use, as principal features, a greater number of features for the values relating to the colour red than for each of the colours green or blue.

5. Device according to one of claims 3 or 4, wherein

   the coefficients for the colour green are, on average, negative and have a higher absolute value than the other colours;
   the coefficients for the colour green have a lower average than the average of the coefficients for the other colours; and/or
   the coefficients for the colour blue have a higher average than the average of the coefficients for the other colours.

6. Device according to one of claims 3 to 5, wherein the majority of the features correspond to colour levels below the average of the colour levels in the histograms.

7. Device according to one of claims 1 to 6, which comprises a means for estimating the sharpness of the captured image, remote from the means for capturing the liver image, and a means for communicating a sharpness index to the image capture means.

8. Device according to claim 7, wherein the means for estimating the sharpness of the image captured is configured to utilise a Sobel filter.

9. Device according to one of claims 1 to 8, wherein the image processing means comprises a means for selecting an image portion.

10. Device according to one of claims 1 to 9, which also comprises a means for introducing into the donor's body at least one optical window for the image capture means and a light source for illuminating the donor's liver, while maintaining the sterility of the operation area.

11. Device according to one of claims 1 to 10, wherein the processing means is configured to detect at least one reflection on the surface of the liver in the captured image, and to extract from the image at least one area with such a reflection.

12. Device according to one of claims 1 to 11, wherein the means for estimating the health of the liver comprises a means for producing a colour histogram and a means for comparing at least one colour of this histogram with a normalised colour.

13. Device according to one of claims 1 to 12, wherein the means for estimating the health of the liver comprises a means for carrying out a sampling of textures and a means for comparing at least one texture with a reference texture.

14. Device according to one of claims 1 to 13, which also comprises a sterile casing configured to contain the image capture means and comprising a transparent rigid image capture window to be positioned in front of the lens of the image capture means.

15. Method for qualitatively evaluating human livers, **characterised in that** it comprises:

> - a phase of learning coefficients and variables based on normalised histograms of colour channels from a portion of a photograph of a liver; and
> - a phase of qualitatively evaluating human livers, which comprises:
>
>> - a step of automatically generating a cropping mask for the liver, by processing colours and/or contours in the image captured, configured to detect at least one reflection on the surface of the liver, in the captured image, and to extract from the image at least one area with such a reflection;
>>
>> - a step of applying the cropping mask to the captured image; and
>> - a step (301) of computing normalised histograms of colour channels from a cropped portion of a photograph of a liver;
>> - a step (304) of loading coefficients obtained from the learning phase;
>> - a step (305) of extracting values from the histograms corresponding to variables selected on completion of the learning phase;
>> - a step (306) of calculating a linear combination of the assigned extracted values of the loaded coefficients; and
>> - a step (308, 309) of displaying an item of information representative of the result of the linear combination calculation.

20

22  23

21

29

28

Figure 1

42

40

41

43

45

50

44

48  49

46

47

51

52

Figure 2

Figure 3

110

| Insertion du capteur d'image dans une housse | 111 |

| Ouverture d'application et connexion | 112 |

| Capture d'image avec flash et éventuelle mire | 113 |

| Extraction d'image de foie | 114 |

| Création de répertoire et documentation | 115 |

| Transmission de l'image extraite | 116 |

| Segmentation de l'image en sous-parties | 117 |

| Classification et estimation de taux de stéatose | 118 |

| Comparaison avec des valeurs limites | 119 |

| Réception et mémorisation des résultats | 120 |

| Retrait de housse | 121 |

Figure 4

Figure 5

Figure 6

Figure 7

Figure 8

Figure 9

Figure 10

Figure 11

Figure 12

Figure 13

Coefficients de modèle (x 10⁻⁴)

Figure 14

Figure 15

Diapo 10 « Prédiction »

Figure 16

Figure 17

Figure 18

Calcul d'histogrammes — 301

Lissage — 302

Normalisations — 303

Chargement des coefficients Bk issus de l'apprentissage — 304

Extraction des valeurs correspondant aux variables — 305

Calcul de y — 306

Normalisation de résultat — 307

Affichage de valeur de y et d'écart-type — 308

Affichage de classe de foie — 309

Figure 19

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SARA MOCCIA**. *Computer-assisted liver-graft steatosis assessment via learning-based texture analysis*
- Analyse en composantes principales. **GILBERT SAPORTA**. Probabilités, Analyse des données et statistique. 2011
- **TREVOR HASTIE** ; **ROBERT TIBSHIRANI** ; **JEROME FRIEDMAN**. The Elements of Statistical Learning: Data Mining, Inference, and Prediction. Springer, 2009
- **M. JIU** ; **N. PUSTELNIK** ; **S. JANAQI** ; **M. CHEBRE** ; **P. RICOUX**. Sparse hierarchical interaction learning with epigraphical projection. *Journal of Signal Processing Systems*, 2019